# FASCICULE DE BREVET EUROPEEN

(11) **EP 2 662 307 B1**
(45) Date de publication et mention de la délivrance du brevet: **06.01.2016**
(21) Numéro de dépôt: 13162042.9
(22) Date de dépôt: 22.08.2011
(51) Int. Cl.: B65D 75/38, B65D 79/02, B65D 81/20, A61J 1/14

(54) **Détection de l'intégrité d'une poche souple, fermée, étanche, en matière plastique, destinée à recevoir et protéger un produit ou un dispositif biopharmaceutique.**
Erkennung der Unversehrtheit einer dichten, geschlossenen, flexiblen Tasche aus Plastik zur Aufnahme und zum Schutz eines biopharmazeutischen Produkts oder einer biopharmazeutischen Vorrichtung
Detection of the integrity of a tight, closed, soft plastic pouch for receiving and protecting a biopharmaceutical product or a biopharmaceutical device.

(30) Priorité: 30.09.2010 FR 1057930
(43) Date de publication de la demande: 13.11.2013
(62) Demande divisionnaire de: 11758531.5
(73) Titulaire: SARTORIUS STEDIM FMT SAS, 13400 Aubagne (FR)
(72) Inventeur: Böttcher, Lars, 34212 Melsungen (DE); Dahlberg, Martin, 37120 Bovenden (DE)
(74) Mandataire: Derambure, Christian

(56) Documents cités:
- FR-A1- 2 822 445
- GB-A- 2 056 950
- US-A- 5 219 524
- US-A1- 2009 123 332

## Description

L'invention concerne la détection de l'intégrité d'une poche souple, fermée, étanche, en matière plastique, destinée à recevoir et protéger un produit ou un dispositif biopharmaceutique, telle qu'une poche stérile.

L'invention vise, plus spécialement, une poche à témoin d'intégrité / non intégrité, une telle poche qui est intègre, , le procédé de réalisation d'une telle poche à témoin d'intégrité / non intégrité, le procédé d'obtention d'une poche *stricto sensu* intègre, et, enfin, un processus de mise en oeuvre d'une poche *stricto sensu* qui, intègre, est destinée à recevoir et protéger un produit ou un dispositif biopharmaceutique.

On entend ici par produit biopharmaceutique, un produit issu de la biotechnologie - milieux de cultures, cultures cellulaires, solutions tampon, liquides de nutrition artificielle, ou un produit pharmaceutique ou plus généralement un produit destiné à être utilisé dans le domaine médical. On entend par dispositif biopharmaceutique, un dispositif destiné à être mis en oeuvre dans un processus du domaine biologique ou pharmaceutique - moyens de mesure ou de contrôle, moyens de traitement de produit biopharmaceutique, conteneurs ou parties de conteneurs, moyens de transfert de ou de fermeture. De tels produits ou dispositifs biopharmaceutiques sont de haute valeur ajoutée et il importe que l'on s'assure de leur intégrité, notamment de l'absence de toute contamination.

Aux fins de stockage, de transport ou de réalisation d'un certain processus de nature physique, chimique ou biologique, il est usuel de placer de tels produits ou dispositifs biopharmaceutiques dans des poches à usage unique souples, fermées, étanches, stériles, en matière plastique telle que le polyéthylène ou un complexe comprenant du polyéthylène. Une telle poche comporte des moyens d'introduction dans son espace intérieur et des moyens d'extraction depuis son espace intérieur du produit ou dispositif biopharmaceutique, aptes à être amenés, selon les moments et les besoins à l'état ouvert ou à l'état fermé.

On connaît de telles poches dont les deux grandes parois sont directement réunies l'une à l'autre. Une fois expansées, de telles poches ont un volume limité et restent relativement peu épaisses, ce qui justifie qu'on les appelle souvent poches « pillow », ou poches « 2D » (D signifiant dimensions). On connaît aussi des poches 3D qui comportent deux grandes parois reliées par et soudées à deux goussets latéraux, pouvant être pliées à plat ou dépliées déployées, le volume pouvant être alors de 50 litres au moins, jusqu'à 3.000 litres, voire plus. De telles poches 3D sont décrites dans le document WO00/04131 ou commercialisées sous la marque FLEXEL^{®} 3D.

Pour pouvoir recevoir et protéger efficacement un produit ou dispositif biopharmaceutique tel que précédemment défini, il est essentiel que l'espace intérieur de la poche - dite ici poche *stricto sensu-* et son contenu éventuel soient préservés d'une possible quelconque détérioration d'origine extérieure à la poche *stricto sensu,* telle qu'une contamination. Cette préservation est ici désignée par « étanchéité ». La poche *stricto sensu* doit donc être étanche ou du moins présenter un degré d'étanchéité considéré comme satisfaisant. Une telle poche *stricto sensu* est donc conçue et fabriquée pour avoir l'étanchéité requise. Mais, des défauts de fabrication de la poche *stricto sensu* affectant son étanchéité ne peuvent être exclus. Et il se peut d'autre part que l'étanchéité d'une poche *stricto sensu* originellement étanche soit affectée après sa fabrication.

L'étanchéité de la poche *stricto sensu* est affectée dès lors que la poche *stricto sensu* comporte un ou plusieurs passages traversant l'enveloppe qu'elle forme, y compris une porosité, ce ou ces passages ayant un ou plusieurs débouchés sur la face externe de la poche *stricto sensu,* y compris les soudures entre les pièces constitutives de la poche *stricto sensu* en question.

Avant d'utiliser ou envisager d'utiliser une telle poche *stricto sensu,* il est donc indispensable de pouvoir s'assurer qu'elle présente l'étanchéité requise. Une telle poche *stricto sensu* présentant cette étanchéité est dite intègre et est apte à recevoir et protéger un produit ou dispositif biopharmaceutique. Une telle poche *stricto sensu* ne présentant pas cette étanchéité est dite non intègre et, étant inapte à recevoir et protéger un produit ou dispositif biopharmaceutique, doit être écartée et non pas utilisée.

On connaît des procédés et dispositifs de contrôle d'intégrité de poches mis en oeuvre en ligne sur toutes les poches lors de leur fabrication. Ainsi, la norme F 2095 - 01 de ASTM International dont le titre est « Standard Test Methods for Pressure Decay Leak Test for Nonporous Flexible Packages With and Without Restraining Plates » porte plus précisément sur le procédé dit à chute de pression. Ce procédé est envisagé selon deux modes d'exécution : avec ou sans plaques de limitation d'expansion (par exemple le dispositif connu sous la marque SARTOCHECK^{®} 4). Dans tous les cas, de tels dispositifs sont complexes et volumineux et difficilement transportables ou utilisables sur les lieux d'utilisation finale des poches.

D'autre part, à supposer la poche intègre à la fabrication, une perte ou rupture accidentelle de l'intégrité peut se produire après la fabrication et avant l'utilisation finale, par exemple dans les phases de stockage, transport, distribution, livraison ou encore dans la phase qui précède immédiatement la mise en oeuvre de la poche. Ce risque est d'autant plus grand que telle ou telle de ces phases est longue (par exemple la phase de stockage peut atteindre plusieurs années) ou qu'elle implique une manipulation de la poche ou son contact avec des objets propice à son endommagement. Or, une telle non intégrité ne peut être décelé par un procédé de contrôle d'intégrité mis en oeuvre sur le seul lieu de fabrication de la poche, avant que n'intervienne la rupture d'intégrité.

Le document US 6892567 et US 6196056 décrivent des procédés et des appareils pour déterminer l'intégrité d'un emballage ou d'un compartiment reposant sur la transmission d'un gaz test à travers l'emballage ou de la paroi du compartiment. Cet appareil et les instruments de mesure associés forment un ensemble complexe et volumineux.

Le document US 2007/0220956 décrit un procédé et un appareil pour détecter une fuite d'une poche destinée à la réalisation d'un processus biologique et contenant un fluide conducteur, placée dans un conteneur extérieur rigide, consistant en un détecteur de fuite à fonctionnement électrique. Un tel procédé et appareil présentent les mêmes les limites mentionnées précédemment, outre qu'ils ne trouvent application que dans le cas d'une poche remplie, d'un fluide conducteur et enfin de la présence d'un conteneur extérieur rigide.

Le document US 4098577 décrit un procédé et un indicateur pour détecter la perte d'intégrité d'un emballage transparent contenant un produit qui est scellé à l'emballage. L'emballage est empli avec une atmosphère artificielle telle que le gaz carbonique ou l'azote. Un détecteur sensible au pH est placé dans l'emballage et est visible à travers lui. Le détecteur change de couleur en cas de perte de l'atmosphère artificielle par suite d'une perte d'intégrité de l'emballage. Dans les réalisations envisagées, l'emballage est rigide, le produit est un objet solide et le détecteur est écarté de ce dernier. Ce procédé est inadapté dans le cas d'un emballage destiné à recevoir un produit fluide venant au contact du détecteur. En outre, il n'est ni décrit ni suggéré, bien au contraire, que la détection intervienne avant que le produit ne soit placé dans l'emballage.

Le document US 4813541 décrit un procédé et un emballage à témoin d'inviolabilité. L'emballage comprend un premier conteneur interne, rigide, hermétiquement fermé empli d'une première atmosphère et contenant une substance et un second conteneur externe, rigide, hermétiquement fermé dans lequel se trouve - en position espacé grâce à des entretoises - le premier conteneur, la cavité interne du second conteneur extérieure au premier conteneur étant emplie d'une seconde atmosphère à une pression différente de celle de la première atmosphère, et des moyens de détection étant placés dans la cavité et étant sensibles à une modification de la seconde atmosphère due à une rupture d'intégrité du premier conteneur ou du second conteneur. Le document US 4813541, qui décrit une structure très particulière ne suggère pas que la détection intervienne avant que la substance ne soit placée dans le premier conteneur.

Le document WO 01/04624 décrit un système colorimétrique comprenant un détecteur sensible au gaz carbonique.

Le document WO96/12659 décrit un emballage dit inviolable comprenant une membrane intérieure délimitant un compartiment intérieur, lequel contient un premier milieu composé d'air, ainsi qu'une membrane extérieure délimitant un compartiment extérieur qui entoure le compartiment interne et contient un second milieu composé de dioxyde de carbone. Une languette indicatrice, sensible au milieu environnant est disposée à l'intérieur du compartiment extérieur et elle peut donner une indication relative à un changement dans le premier et dans le second milieu.

Le document US 4434893 décrit un emballage pour recevoir des produits comprenant des étuis interne et externe. Dans un mode de réalisation, l'étui interne et l'étui externe ne présentent respectivement qu'une seule paroi flexible et ces étuis interne et externe sont mis sous pression à l'aide d'un gaz présentant une pression supérieure à la pression atmosphérique de manière à gonfler leur paroi flexible. Dans un autre mode de réalisation, les deux parois de chacun des étuis interne et externe sont flexibles. Les produits - capsules, gélules ou analogues - à protéger sont inclus à l'intérieur de l'étui interne et l'étui interne est lui-même localisé à de l'étui externe de manière à former une protection empêchant l'accès aux produits.

Le document US 4436203 décrit un emballage comprenant un containeur interne de taille prédéfinie, empli d'un produit et mis sous pression avant d'être disposé à l'intérieur d'un containeur externe. Après que sa pression interne n'ait été abaissée au-dessous de la pression atmosphérique, ledit containeur externe est fermé. Ainsi, lorsqu'une des parois de l'emballage se perce, le consommateur est alerté du fait que le produit a pu être touché.

Le document FR 2252 619 décrit un dispositif destiné à être utilisé avec un emballage fermé normalement de façon hermétique. Il comporte un détecteur disposé de sorte qu'il soit en communication avec l'intérieur de l'emballage mais également visible de l'extérieur. Ce détecteur contient un colorant sensible au pH, qui présente une première couleur à un pH atmosphérique normal, et une seconde couleur à un pH légèrement supérieur ou inférieur au pH atmosphérique normal.

Le document FR-A-2822445 porte sur un emballage de prélèvements infectieux du type comprenant un sac en matière plastique étanche destiné à contenir un récipient contenant ledit prélèvement logé dans une nappe d'un tissu absorbant **caractérisé en ce que** ledit tissu est coloré et est tel que toute trace d'humidité modifie sa couleur initiale, le sac en matière plastique étant transparent.

Le document US 5219 524 porte sur un système de préservation d' un article contenant de l'acide qui est en contact avec un matériau substrat alcalines et une atmosphère artificielle qui est sensiblement exempte d'oxygène gazeux. L'article, matériau substrat alcalin et de l'atmosphère artificielle sont hermétiquement fermés dans un conteneur, au moins une partie du conteneur étant au moins partiellement transparent afin de permettre la visualisation de l'article de l'extérieur du conteneur.

L'invention a donc pour but de pallier les problèmes précédemment mentionnés et de permettre d'identifier l'intégrité ou la non intégrité d'une poche *stricto sensu* à tout moment souhaité après sa fabrication et au moins juste avant son utilisation projetée, et cela de façon sûre, aisée, rapide, sans la nécessité d'un dispositif dédié lourd ou complexe, ou d'opérations difficiles ou délicates, et sans la nécessité de devoir recourir à un personnel spécialisé spécialement et exclusivement dédié au contrôle de l'intégrité, et sans la nécessité d'avoir à tester la poche de façon positive pour s'assurer de son intégrité, la non intégrité étant révélée de façon automatique, et donc sans la nécessité, lorsque la poche fait partie d'une pluralité de poches, d'avoir à tester chaque poche de façon positive.

A cet effet, selon un premier aspect, l'invention a pour objet une poche à témoin d'intégrité / non intégrité en vue de recevoir et protéger un produit ou dispositif biopharmaceutique, comprenant :
▪ une première enveloppe, intérieure, souple, fermée, étanche, en matière plastique, comportant une paroi délimitant un premier espace, formant *stricto sensu* une poche destinée à recevoir le produit ou dispositif biopharmaceutique, comportant des premiers moyens d'introduction et des premiers moyens d'extraction du produit ou dispositif se trouvant à l'état fermé,
   ■ une seconde enveloppe, extérieure, fermée, étanche, en matière plastique, comportant une paroi délimitant un second espace dans lequel se trouve la première enveloppe / poche *stricto sensu,* comportant des seconds moyens d'introduction et des seconds moyens d'extraction de la première enveloppe / poche *stricto sensu,* se trouvant à l'état fermé, un espace intermédiaire étant ainsi ménagé dans le second espace hors de la première enveloppe / poche *stricto sensu,*
   ■ des moyens espaceurs interposés entre la paroi et la paroi aptes à ce que la face intérieure de la paroi n'occulte pas un éventuel débouché de non intégrité sur la face externe de la paroi,
   ■ au moins un gaz traceur, se trouvant soit dans le premier espace soit dans l'espace intermédiaire, à une pression partielle différente de celle de l'espace intermédiaire ou du premier espace, respectivement, introduit dans le premier espace et/ou l'espace intermédiaire par des moyens d'introduction de gaz traceur, se trouvant à l'état fermé,
▪ au moins une couche continue de détection colorimétrique du gaz traceur, responsive à la concentration du gaz traceur qui l'atteint en passant d'une première couleur à une seconde couleur différente, faisant partie intégrante d'une paroi de la première enveloppe / poche *stricto sensu* et/ou d'une paroi de la seconde enveloppe et/ou des moyens espaceurs,
■ la disposition des parois de la première enveloppe / poche *stricto sensu* et de la seconde enveloppe, des moyens espaceurs, du gaz traceur et de la couche de détection colorimétrique, d'une part, la perméabilité ou l'imperméabilité au gaz traceur de la couche de détection colorimétrique, et des moyens espaceurs, d'autre part, étant choisies pour que la couche de détection colorimétrique ne puisse pas être atteinte par du gaz traceur se trouvant dans l'espace intermédiaire ou dans le premier espace avec une concentration passant la valeur de transition, avec la première enveloppe / poche *stricto sensu* intègre, et que la couche de détection colorimétrique soit nécessairement atteinte par du gaz traceur se trouvant dans l'espace intermédiaire ou dans le premier espace avec une concentration passant la valeur de transition, avec une première enveloppe / poche *stricto sensu* non intègre.

Ainsi, si la couche de détection colorimétrique est identifiée de la première couleur, la première enveloppe / poche *stricto sensu* est considérée comme intègre et apte à recevoir et protéger le produit ou dispositif biopharmaceutique, alors que si elle est identifiée de la seconde couleur, elle est considérée comme non intègre et inapte à recevoir et protéger le produit ou dispositif biopharmaceutique.

Selon les réalisations, les moyens espaceurs comprennent au moins une couche poreuse recouvrant intégralement fonctionnellement la face externe de la paroi de la première enveloppe / poche *stricto sensu* et/ou la face intérieure de la paroi de la seconde enveloppe et/ou la face de la couche de détection colorimétrique tournée vers l'espace intermédiaire ou bien les moyens espaceurs comprennent au moins une couche poreuse qui soit recouvre structurellement la face externe de la paroi de la première enveloppe / poche *stricto sensu* et/ou la face intérieure de la paroi de la seconde enveloppe et/ou la face de la couche de détection colorimétrique tournée vers l'espace intermédiaire, soit est placée dans l'espace intermédiaire.

Selon les réalisations, le gaz traceur est dans le premier espace à une pression partielle plus grande que dans l'espace intermédiaire, la couche de détection colorimétrique passant de la première couleur à la seconde couleur en réponse au passage de la concentration en gaz traceur qui l'atteint au-dessus d'une valeur de transition, soit le gaz traceur est dans le premier espace à une pression partielle plus petite que dans l'espace intermédiaire, la couche de détection colorimétrique passant de la première couleur à la seconde couleur en réponse au passage de la concentration en gaz traceur qui l'atteint au-dessous d'une valeur de transition.

Selon une première famille de réalisations, une couche de détection colorimétrique est perméable au gaz traceur et fait partie intégrante de la paroi de la première enveloppe / poche *stricto sensu,* étant soit située sur la face externe et dans l'espace intermédiaire, soit située sur la face interne et dans le premier espace, soit insérée dans la paroi, interposée entre sa face externe et sa face interne et entre le premier espace et l'espace intermédiaire.

Selon une deuxième famille de réalisations, une couche de détection colorimétrique fait partie intégrante de la paroi de la seconde enveloppe, étant soit située sur la face intérieure et dans l'espace intermédiaire, soit insérée dans la paroi, interposée entre la face intérieure et sa face extérieure, la couche de la paroi de la seconde enveloppe adjacente d'un coté à la couche de détection colorimétrique et de l'autre coté à la face intérieure étant alors perméable au gaz traceur.

Selon une troisième famille de réalisations, une couche de détection colorimétrique fait partie intégrante d'une couche poreuse des moyens espaceurs étant soit située vers la face externe de la paroi de la première enveloppe / poche *stricto sensu,* soit située vers la face intérieure de la seconde enveloppe ou bien une couche de détection colorimétrique et une couche poreuse des moyens espaceurs sont structurellement confondues pour former une seule même couche.

Les réalisations selon ces différentes familles peuvent, le cas échéant, être combinées entre elles.

Selon les cas, la première enveloppe / poche *stricto sensu* est une poche 2D, ou une poche 3D comportant deux goussets. Dans ce dernier cas, et selon une réalisation, les moyens espaceurs comprennent au moins une couche poreuse recouvrant intégralement fonctionnellement, et le cas échéant structurellement, la face externe de l'intérieur des goussets.

Selon des réalisations possibles, les moyens espaceurs comprennent au moins une couche poreuse recouvrant intégralement fonctionnellement, et le cas échéant structurellement, les premiers moyens d'introduction et les premiers moyens d'extraction du produit ou dispositif biopharmaceutique et les moyens d'introduction de gaz traceur de la première enveloppe / poche *stricto sensu*. Ou bien, les moyens espaceurs comprennent au moins une couche poreuse en tissu, en non tissé, en PE, en PP, en PTFE.

Selon des réalisations possibles, un gaz traceur est choisi dans le groupe comprenant l'oxygène, le gaz carbonique, l'hélium.

Selon deux réalisations possibles, l'un du premier espace ou de l'espace intermédiaire contient du gaz traceur et que l'autre de l'espace intermédiaire ou du premier espace ne contient pas ou substantiellement pas de gaz traceur.

Selon une réalisation, la seconde enveloppe est apte à permettre d'identifier depuis l'extérieur d'elle-même si la couche de détection colorimétrique est de la première couleur ou de la seconde couleur.

Selon une réalisation, la seconde enveloppe est souple.

Selon une réalisation, la poche à témoin d'intégrité / non intégrité comporte en outre un suremballage extérieur de protection dans lequel est logée la seconde enveloppe, dans laquelle se trouve la première enveloppe / poche *stricto sensu*.

Selon un deuxième aspect, l'invention a pour objet une poche intègre à témoin d'intégrité / non intégrité constituée par une poche à témoin d'intégrité / non intégrité telle qu'elle a été décrite précédemment, dont la couche de détection colorimétrique est de la première couleur.

Selon un troisième aspect, l'invention a pour objet un procédé de réalisation d'une poche à témoin d'intégrité / non intégrité telle qu'elle a été décrite précédemment, dans lequel :
■ on dispose d'une première enveloppe / poche *stricto sensu,* d'une seconde enveloppe dont les seconds moyens d'introduction de la première enveloppe / poche *stricto sensu* sont à l'état ouvert, de moyens espaceurs, du gaz traceur, la couche de détection colorimétrique faisant partie intégrante d'une paroi de la première enveloppe / poche *stricto sensu* et/ou d'une paroi de la seconde enveloppe et/ou des moyens espaceurs,
■ on introduit la première enveloppe / poche *stricto sensu* dans le second espace de la seconde enveloppe via les seconds moyens d'introduction à l'état ouvert, les moyens espaceurs étant interposés entre la paroi et la paroi de sorte que la face intérieure de la paroi n'occulte pas un éventuel débouché de non intégrité sur la face externe de la paroi,
■ on introduit le gaz traceur, soit dans le premier espace soit dans l'espace intermédiaire, à une pression partielle différente de celle de l'espace intermédiaire ou du premier espace, respectivement, via les moyens d'introduction de gaz traceur, se trouvant à l'état ouvert, puis on amène ces moyens d'introduction à l'état fermé,
■ on choisit l'agencement pour que la couche de détection colorimétrique ne puisse pas être atteinte ou au contraire soit nécessairement atteinte par du gaz traceur, comme exigé et on choisit en conséquence la perméabilité ou l'imperméabilité au gaz traceur de la couche de détection colorimétrique et des moyens espaceurs pour que la couche de détection colorimétrique soit effectivement atteinte par du gaz traceur lorsque cela est nécessaire,
■ la première enveloppe / poche *stricto sensu* et la seconde enveloppe étant fermées.

Selon un quatrième aspect, l'invention a pour objet un procédé d'obtention d'une poche *stricto sensu* intègre et prête à recevoir et protéger un produit ou dispositif biopharmaceutique, dans lequel :
▪ on dispose d'une poche à témoin d'intégrité / non intégrité telle qu'elle a été décrite précédemment,
▪ on identifie si la couche de détection colorimétrique est de la première couleur ou de la seconde couleur,
■ si la couche de détection colorimétrique est identifiée de la première couleur :
   ∘ on amène les seconds moyens d'extraction de la première enveloppe / poche *stricto sensu* dont est pourvue la seconde enveloppe à l'état ouvert,
   ∘ on extrait la première enveloppe / poche *stricto sensu* du second espace de la seconde enveloppe,
   o et on amène les premiers moyens d'introduction du produit ou dispositif biopharmaceutique dont est pourvu la première enveloppe / poche *stricto sensu* considérée comme intègre, à l'état ouvert en vue d'y placer le produit ou dispositif biopharmaceutique,
■ alors que si la couche de détection colorimétrique est identifiée de la seconde couleur, on ne retient pas la première enveloppe / poche *stricto sensu* en vue d'y placer le produit ou dispositif biopharmaceutique, la première enveloppe / poche *stricto sensu* étant considérée comme non intègre.

Selon un cinquième aspect, l'invention a pour objet un processus de mise en oeuvre d'une poche *stricto sensu* qui, intègre, est destinée à recevoir et protéger un produit ou dispositif biopharmaceutique, comprenant :
■ sur un lieu de fabrication, des opérations initiales consistant à fabriquer une poche *stricto sensu,*
■ sur un lieu d'utilisation, des opérations finales consistant à utiliser la poche *stricto sensu* en y plaçant le produit ou dispositif biopharmaceutique,
■ sur un ou plusieurs lieux, des opérations intermédiaires de stockage, transport, manipulation, et comprenant :
   ▪ la réalisation, sur le lieu de fabrication, d'une poche à témoin d'intégrité / non intégrité comprenant une première enveloppe / poche *stricto sensu,* par le procédé de réalisation tel qu'il a été décrit précédemment,
   ▪ le transport de la poche à témoin d'intégrité / non intégrité du lieu de fabrication au lieu d'utilisation, moyennant le cas échéant des opérations de stockage et de manipulation,
   ▪ et, au moins sur le lieu d'utilisation et avant d'extraire la première enveloppe / poche *stricto sensu* de la seconde enveloppe en vue d'y placer le produit ou dispositif biopharmaceutique, l'identification que la couche de détection colorimétrique est de la première couleur ou de la seconde couleur,
   ■ si la couche de détection colorimétrique est identifiée de la première couleur, l'utilisation de la première enveloppe / poche *stricto sensu* considérée comme intègre en vue d'y placer le produit ou dispositif biopharmaceutique pour le recevoir et le protéger,
   ■ alors que si la couche de détection colorimétrique est identifiée de la seconde couleur, la non utilisation de la première enveloppe / poche *stricto sensu* considérée comme non intègre en vue d'y placer le produit ou dispositif biopharmaceutique.

Selon une réalisation, l'identification que la couche de détection colorimétrique est de la première couleur ou de la seconde couleur est réalisée en temps masqué lors des opérations intermédiaires de stockage, transport, manipulation.

Selon une réalisation, l'identification que la couche de détection colorimétrique est de la première couleur ou de la seconde couleur est réalisée sur le lieu d'utilisation juste avant d'extraire la première enveloppe / poche *stricto sensu* de la seconde enveloppe en vue d'y placer le produit ou dispositif biopharmaceutique.

On décrit maintenant plusieurs modes de réalisation de l'invention à l'aide des dessins, dans lesquels :

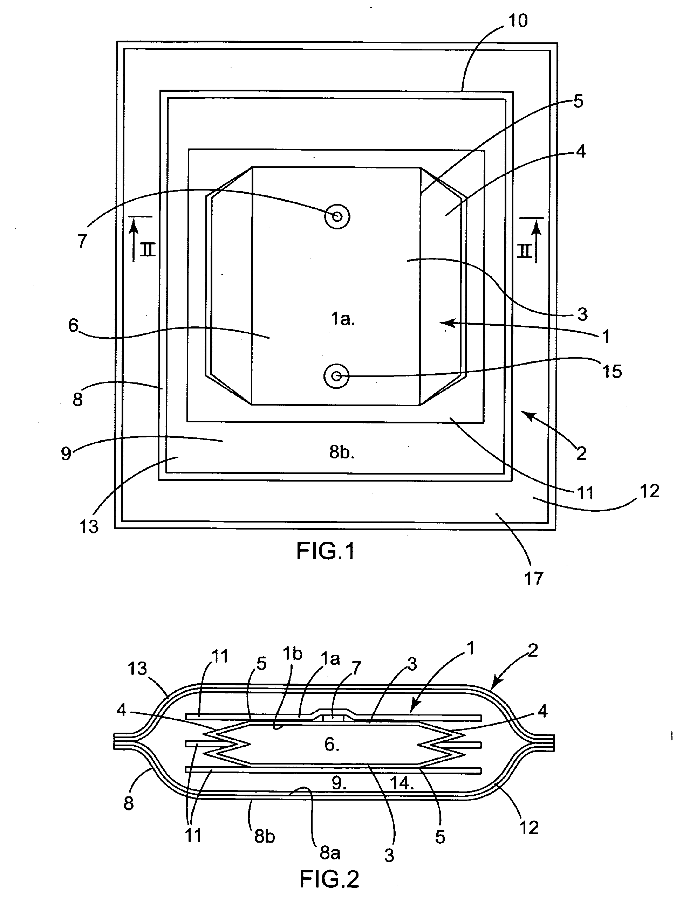

- La figure 1 est une vue de dessus d'une poche à témoin d'intégrité / non intégrité du type dans lequel la poche *stricto sensu* est de type 3D et la poche à témoin d'intégrité / non intégrité logée dans un suremballage extérieur de protection, cette poche étant représentée pliée à plat telle qu'elle se présente par exemple lors de son stockage ou de son transport.
- La figure 2 est une vue en coupe transversale de la poche à témoin d'intégrité / non intégrité de la figure 1, sans le suremballage extérieur de protection, dans une réalisation possible et non limitative.

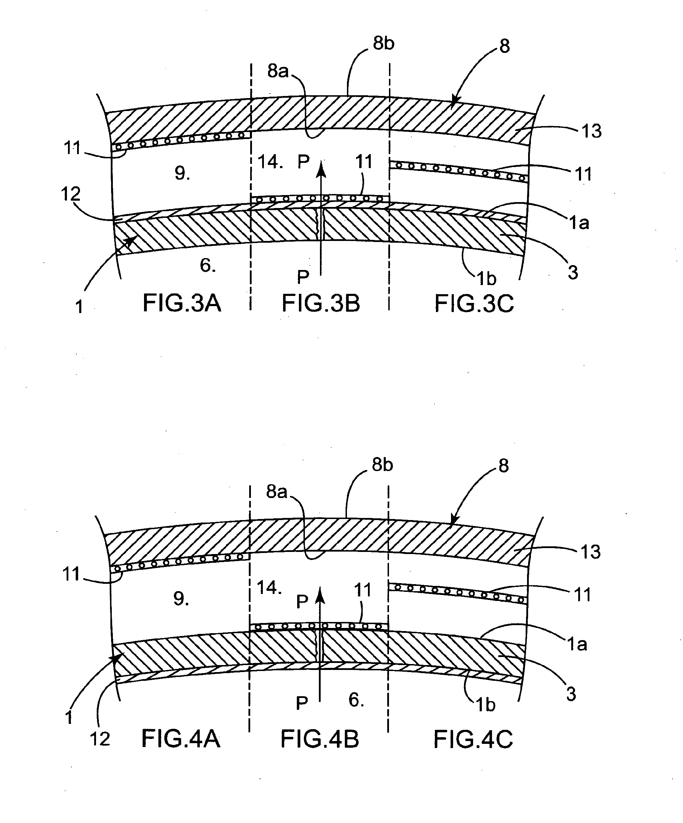

- Les figures 3A, 3B et 3C sont trois vues en coupe transversale, partielles, à plus grande échelle, illustrant trois réalisations possibles de la poche à témoin d'intégrité / non intégrité, dans lesquelles la couche de détection colorimétrique est située vers la face externe de la paroi de la première enveloppe / poche *stricto sensu* dont elle fait partie intégrante, les moyens espaceurs étant, respectivement, associés à la face intérieure de la paroi de la seconde enveloppe (figure 3A), associés à la paroi de la première enveloppe / poche *stricto sensu* vers sa face externe (figure 3B), situés entre les parois de la première enveloppe / poche *stricto sensu* et de la seconde enveloppe (figure 3C).

- Les figures 4A, 4B et 4C sont trois vues en coupe transversale, partielles, à plus grande échelle, illustrant trois réalisations possibles de la poche à témoin d'intégrité / non intégrité, dans lesquelles la couche de détection colorimétrique est située vers la face interne de la paroi de la première enveloppe / poche *stricto sensu* dont elle fait partie intégrante, les moyens espaceurs étant, respectivement, associés à la face intérieure de la paroi de la seconde enveloppe (figure 4A), associés à la face externe de la paroi de la première enveloppe / poche *stricto sensu* (figure 4B), situés entre les parois de la première enveloppe / poche *stricto sensu* et de la seconde enveloppe (figure 4C).

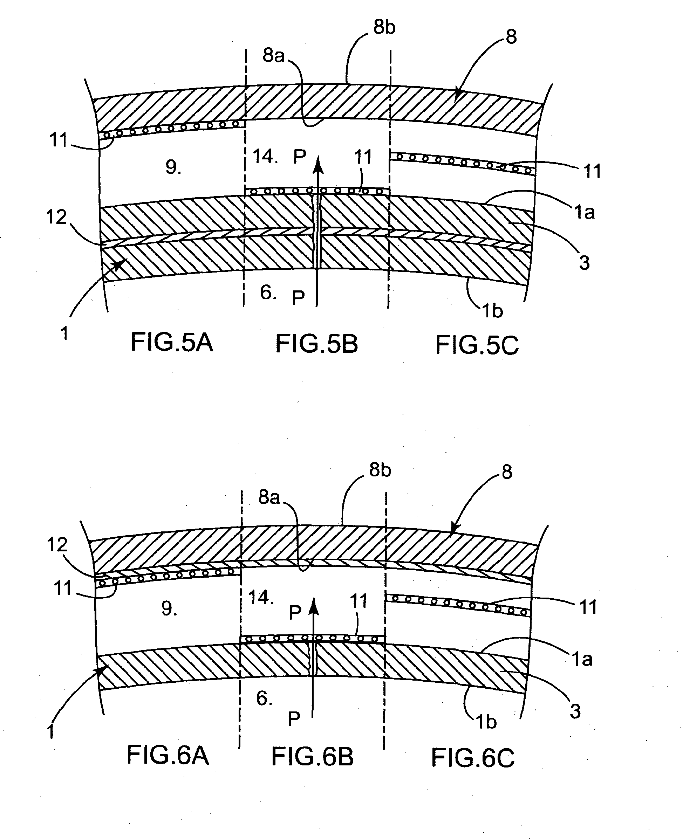

- Les figures 5A, 5B et 5C sont trois vues en coupe transversale, partielles, à plus grande échelle, illustrant trois réalisations possibles de la poche à témoin d'intégrité / non intégrité, dans lesquelles la couche de détection colorimétrique est insérée dans la paroi de la première enveloppe / poche *stricto sensu* dont elle fait partie intégrante, les moyens espaceurs étant, respectivement, associés à la face intérieure de la paroi de la seconde enveloppe (figure 5A), associés à la face externe de la paroi de la première enveloppe / poche *stricto sensu* (figure 5B), situés entre les parois de la première enveloppe / poche *stricto sensu* et de la seconde enveloppe (figure 5C).
- Les figures 6A, 6B et 6C sont trois vues en coupe transversale, partielles, à plus grande échelle, illustrant trois réalisations possibles de la poche à témoin d'intégrité / non intégrité, dans lesquelles la couche de détection colorimétrique est située vers la face intérieure de la paroi de la seconde enveloppe dont elle fait partie intégrante, les moyens espaceurs étant, respectivement, associés à la paroi de la seconde enveloppe vers sa face intérieure (figure 6A), associés à la face externe de la paroi de la première enveloppe / poche *stricto sensu* (figure 6B), situés entre les parois de la première enveloppe / poche *stricto sensu* et de la seconde enveloppe (figure 6C).

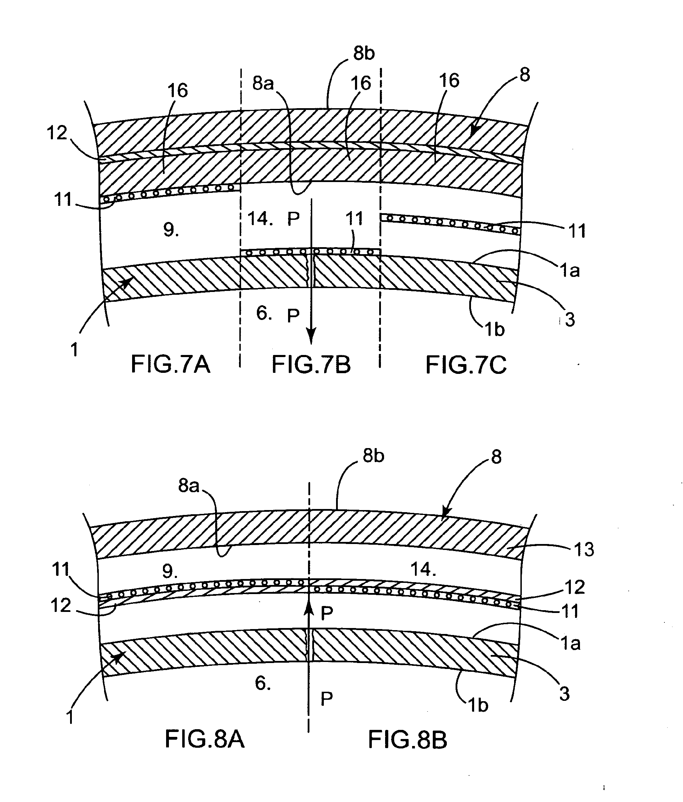

- Les figures 7A, 7B et 7C sont trois vues en coupe transversale, partielles, à plus grande échelle, illustrant trois réalisations possibles de la poche à témoin d'intégrité / non intégrité, dans lesquelles la couche de détection colorimétrique est insérée dans la paroi de la seconde enveloppe dont elle fait partie intégrante, les moyens espaceurs étant, respectivement, associés à la face intérieure de la paroi de la seconde enveloppe (figure 7A), associés à la face externe de la paroi de la première enveloppe / poche *stricto sensu* (figure 7B), situés entre les parois de la première enveloppe / poche *stricto sensu* et de la seconde enveloppe (figure 7C).
- Les figures 8A et 8B sont deux vues en coupe transversale, partielles, à plus grande échelle, illustrant trois réalisations possibles de la poche à témoin d'intégrité / non intégrité, dans lesquelles la couche de détection colorimétrique fait partie intégrante d'une couche poreuse des moyens espaceurs, la couche de détection colorimétrique étant située vers la face externe de la première enveloppe / poche *stricto sensu* paroi (figure 8A), située vers la face intérieure de la seconde enveloppe (figure 8B).

Une poche 3D 1 souple, fermée (pendant les phases où cela est souhaité), étanche dans son ensemble, stérile, en matière plastique, notamment à usage unique, destinée dans le domaine biopharmaceutique à recevoir et protéger aux fins de stockage, de transport ou de réalisation d'un certain processus de nature physique, chimique ou biologique, un produit ou un dispositif biopharmaceutique est connue de l'état de la technique (voir par exemple le document WO00/04131 et la poche connue sous la marque FLEXEL^{®} 3D).

Pour pouvoir recevoir et protéger efficacement le produit ou dispositif biopharmaceutique, l'espace intérieur de la poche 1 et son contenu éventuel doivent être préservés d'une possible quelconque détérioration d'origine extérieure à la poche 1, telle qu'une contamination. La poche 1 doit donc être étanche au sens où cela a été défini. Une telle poche 1 est conçue et fabriquée pour être étanche, mais, des défauts de fabrication ne peuvent être exclus et l'étanchéité peut être affectée après fabrication.

L'étanchéité est affectée dès lors que la poche 1 comporte un ou plusieurs passages T traversant l'enveloppe qu'elle forme, y compris une porosité, ce ou ces passages ayant un ou plusieurs débouchés D sur la face externe 1a de la poche 1, y compris les soudures 5 entre les pièces constitutives de la poche 1 en question.

Avant d'utiliser ou envisager d'utiliser une telle poche 1, il est donc indispensable de pouvoir s'assurer qu'elle présente l'étanchéité requise. Ainsi qualifiée d'«intègre », la poche 1 est apte à recevoir et protéger un produit ou dispositif biopharmaceutique, alors qu'une poche 1 ne présentant pas cette étanchéité est dite non intègre et, étant inapte à recevoir et protéger un produit ou dispositif biopharmaceutique, doit dans le cadre du processus de mise en oeuvre des poche 1 être écartée et non pas utilisée.

L'invention vise à identifier l'intégrité ou la non intégrité d'une telle poche 1 à tout moment souhaité après sa fabrication et au moins juste avant son utilisation projetée.

Pour cela, on met en oeuvre une poche 2 qui reçoit la poche 1.

Pour distinguer les poches 1 et 2, la poche 1 est qualifiée de « poche *stricto sensu* » alors que la poche 2 est qualifiée de « poche à témoin d'intégrité / non intégrité ».

L'expression « poche *stricto sensu* », vise donc la poche 1 destinée à recevoir et protéger directement le produit ou dispositif biopharmaceutique, laquelle poche *stricto sensu* 1 est incorporée dans l'ensemble objet de l'invention appelé « poche à témoin d'intégrité / non intégrité » 2.

Une poche *stricto sensu* 1 3D peut être de volume important de 50 litres au moins, jusqu'à 1.000 litres, voire même plus. Une telle poche *stricto sensu* 1 comporte typiquement une paroi principale 3 en deux parties reliées de façon fixe et étanche à deux goussets latéraux 4 par des soudures 5.

Une telle poche *stricto sensu* 3D peut être pliée à plat - par exemple pour le stockage, le transport, la manipulation - ou dépliée déployée, lorsqu'elle reçoit un produit ou dispositif biopharmaceutique.

Les parois 3, 4 constitutives de la poche 1 et les soudures 5 étanches forment une enveloppe, appelée ici « première enveloppe », également désignée par la référence 1. Par convention, on entend par « la » paroi 3, 4 aussi bien l'ensemble des parois de la poche *stricto sensu* 1 que chacune d'elles.

La paroi 3, 4 est réalisée à partir d'une ou de plusieurs matières plastique flexibles, dont certaines au moins sont non poreuses de sorte que la poche *stricto sensu* 1 soit étanche dans son ensemble. La paroi 3, 4 peut être monocouche ou multicouche. Elle peut incorporer une matière plastique à haute capacité barrière aux gaz, tel que l'EVOH, cela n'étant qu'exemplatif et non limitatif.

La poche *stricto sensu* 1, à savoir sa paroi 3, 4, comporte une face externe 1a et une face interne 1b. Les termes « externe » et « interne » se comprennent en relation avec ce qui est localisé, respectivement, hors de et dans la poche *stricto sensu* 1. La poche *stricto sensu* 1 délimite à l'intérieur d'elle-même un premier espace 6, dont la limite est la face interne 1b et dont le volume est approprié pour être apte à recevoir le produit ou dispositif biopharmaceutique lorsqu'il est souhaité qu'il soit dans la poche *stricto sensu* 1.

La poche *stricto sensu* 1 comporte des premiers moyens d'introduction 7 associés à la paroi 3, en vue de l'introduction lorsque cela est souhaité, du produit ou dispositif biopharmaceutique dans le premier espace 6. Une telle introduction intervient alors que la poche *stricto sensu* 1 a été préalablement extraite de la poche 2 à témoin d'intégrité / non intégrité. De tels premiers moyens d'introduction 7 peuvent se présenter sous la forme d'un ou de plusieurs passages agencés dans la paroi 3 pour, lorsque nécessaire, être amenés, ou se trouver, à l'état ouvert. De tels passages sont généralement également agencés pour pouvoir être ensuite amenés à l'état fermé, notamment par soudure, une fois le produit ou dispositif biopharmaceutique introduit dans le premier espace 6.

La poche *stricto sensu* 1 comporte également des premiers moyens d'extraction associés à la paroi 3, en vue de l'extraction, lorsque cela est souhaité, du produit ou dispositif biopharmaceutique depuis le premier espace 6. Une telle extraction intervient forcément alors que la poche *stricto sensu* 1 a été antérieurement extraite de la poche 2 à témoins d'intégrité / non intégrité pour l'introduction du produit ou dispositif biopharmaceutique comme il vient d'être décrit. De tels premiers d'extraction peuvent être une déchirure ou analogue irréversible de la paroi 3 de la poche *stricto sensu* 1, laquelle est généralement à usage unique.

Une telle poche *stricto sensu* 1 se trouve typiquement soit dans un état plié à plat, par exemple pour le stockage, le transport, la manipulation soit dans un état déplié déployé pour recevoir et protéger le produit ou dispositif biopharmaceutique, soit encore dans un état intermédiaire. La poche *stricto sensu* 1 peut être déformée pour passer de l'un à l'autre de ces états.

Une telle poche *stricto sensu* 1 peut, selon les nécessités, être associée à un conteneur rigide de contention de la poche1 par l'extérieur. Un conteneur tel que celui considéré est par exemple décrit dans une variante possible de réalisation dans le document EP-A-1012073 ou commercialisé sous la marque PALLETTANK^{®}.

Le processus de mise en oeuvre d'une telle poche *stricto sensu* 1 comprend :
▪ sur un lieu de fabrication, des opérations initiales consistant à fabriquer la poche *stricto sensu* 1,
▪ sur un lieu d'utilisation, le plus souvent différent et distant du lieu de fabrication, des opérations finales consistant à utiliser la poche *stricto sensu* 1 en y plaçant le produit ou dispositif biopharmaceutique aux fins souhaitées
■ et sur un ou plusieurs lieux (fixes ou mobiles), des opérations intermédiaires de stockage, transport, manipulation ou autre, qui peuvent s'étendre sur une période assez longue, par exemple plusieurs mois, voire plusieurs années, s'agissant du stockage.

Afin d'identifier l'intégrité ou la non intégrité de la poche *stricto sensu* 1 à tout moment souhaité après sa fabrication et au moins juste avant son utilisation projetée, le processus de mise en oeuvre des poches *stricto sensu* est modifié, conformément à l'invention.

Ce processus comprend, tout d'abord, la réalisation, sur le lieu de fabrication, d'une poche à témoin d'intégrité 2 comprenant la poche *stricto sensu* 1.

On décrit maintenant une poche 2 à témoin d'intégrité / non intégrité comprenant une poche *stricto sensu* 1 qui est soit une poche 3D, comme il a été précédemment mentionné, soit une poche 2D, laquelle est dépourvue des goussets 4.

La poche 2 à témoin d'intégrité / non intégrité comprend, tout d'abord la poche *stricto sensu* 1, également désignée « première enveloppe » 1.

Dans la poche 2 à témoin d'intégrité / non intégrité, la première enveloppe / poche *stricto sensu* 1 est fermée et vide du produit ou dispositif biopharmaceutique. Ses premiers moyens 7 d'introduction et ses premiers moyens d'extraction se trouvent à l'état fermé.

La poche 2 à témoin d'intégrité / non intégrité comprend ensuite une seconde enveloppe 8, extérieure, la première enveloppe / poche *stricto sensu* 1 étant intérieure et destinée à être placée dans la seconde enveloppe 8.

La seconde enveloppe 8 comporte une paroi 13 ayant le plus souvent plusieurs parties soudées entre elles le long de soudures étanches. On entend par « la » paroi 13 aussi bien la paroi 13 unique que l'ensemble de ces parties de parois ou chacune d'elles s'il y en a plusieurs.

La seconde enveloppe 8 est fermée et étanche. Elle est préférentiellement souple, de sorte à pouvoir être à plat pour le stockage, le transport, la manipulation.
La paroi 13 est réalisée à partir d'une ou de plusieurs matières plastique, flexibles si la seconde enveloppe 8 est souple, non poreuses de sorte que la seconde enveloppe 8 soit étanche dans son ensemble. La paroi 13 peut être monocouche ou multicouche. Elle peut incorporer une matière plastique à haute capacité barrière aux gaz, tel que l'EVOH, cela n'étant qu'exemplatif et non limitatif.

La seconde enveloppe 8, à savoir sa paroi 13, comporte une face intérieure 8a et une face extérieure 8b. Les termes « intérieur » et « extérieur » se comprennent en relation avec ce qui est localisé, respectivement, dans et hors de la seconde enveloppe 8. La seconde enveloppe 8 délimite à l'intérieur d'elle-même un second espace 9, dont la limite est la face intérieure 8a et dont le volume est approprié pour être apte à recevoir la poche *stricto sensu* 1, alors vide du produit ou dispositif biopharmaceutique.

La seconde enveloppe 8 comporte des seconds moyens d'introduction 10 associés à la paroi 13, en vue de l'introduction préalable, lorsque cela est souhaité, de la première enveloppe / poche *stricto sensu* 1 dans le second espace 9. Une telle introduction intervient pour et lors de la réalisation de la poche 2 à témoin d'intégrité / non intégrité. De tels seconds moyens d'introduction 10 peuvent se présenter sous la forme d'un ou de plusieurs passages agencés dans la paroi 13 pour, lorsque nécessaire, être amenés ou se trouver à l'état ouvert et être ensuite amenés à l'état fermé, notamment par soudure, une fois la première enveloppe / poche *stricto sensu* 1 introduite dans le second espace 9.

La seconde enveloppe 8 comporte également des seconds moyens d'extraction associés à la paroi 13, en vue de l'extraction, lorsque cela est souhaité, de la première enveloppe / poche *stricto sensu* 1 depuis le second espace 9. Une telle extraction intervient à l'occasion de, et avant, l'introduction du produit ou dispositif biopharmaceutique dans le premier espace 6. De tels seconds moyens d'extraction peuvent être une déchirure ou analogue irréversible de la paroi 13 de la seconde enveloppe 8, laquelle est généralement à usage unique.

Dans la poche 2 à témoin d'intégrité / non intégrité, la première enveloppe / poche *stricto sensu* 1 se trouve dans le second espace 9 de la seconde enveloppe 8, dont les seconds moyens d'introduction 10 et les seconds moyens d'extraction se trouvent à l'état fermé.

A l'extérieur de la première enveloppe / poche *stricto sensu* 1 mais à l'intérieur de la seconde enveloppe 8 est formé un espace 14 qualifié « d'espace intermédiaire », situé dans le second espace 9 hors de la première enveloppe / poche *stricto sensu* 1. Les limites de l'espace intermédiaire 14 sont la face externe 1a de la première enveloppe / poche *stricto sensu* 1 et la face intérieure 8a de la seconde enveloppe 8.

La poche 2 à témoin d'intégrité / non intégrité comprend ensuite des moyens espaceurs 11, logés dans espace 14, interposés entre la paroi 3, 4 de la première enveloppe / poche *stricto sensu* 1 et la paroi 13 de la seconde enveloppe 8. Ces moyens espaceurs 11 sont aptes à ce que la face intérieure 8a de la paroi 13 de la seconde enveloppe 8 n'occulte pas un éventuel débouché de non intégrité D sur la face externe 1a de la paroi 3, 4 de la première enveloppe / poche *stricto sensu* 1.

Les moyens espaceurs 11 comprennent une ou plusieurs couches poreuses recouvrant intégralement fonctionnellement la face externe 1a de la paroi 3, 4 de la première enveloppe / poche *stricto sensu* 1 et/ou la face intérieure 8a de la paroi 13 de la seconde enveloppe 8 et/ou la face d'une couche de détection colorimétrique 12 tournée vers l'espace intermédiaire 14, sur laquelle on revient par la suite.

Une couche des moyens espaceurs 11 est poreuse par suite soit d'un agencement ménageant une porosité soit de l'emploi d'un ou de plusieurs matériaux poreux. Par exemple, les moyens espaceurs 11 comprennent une ou plusieurs couches poreuses en tissu, en non tissé, en PE, en PP, en PTFE ou autre matériau analogue.

On entend ici par « porosité », le fait que les moyens espaceurs 11 comportent une multiplicité d'interstices adjacents les uns aux autres, traversant d'un coté à l'autre la ou les couches qui constituent les moyens espaceurs 11, de sorte que, d'une part, tout éventuel débouché de non intégrité D sur la face externe 1a de la paroi 3, 4 de la première enveloppe / poche *stricto sensu* 1 se trouve nécessairement au regard de et en communication avec au moins un débouché d'interstice et que, d'autre part, un gaz, tel qu'un gaz traceur sur lequel on revient par la suite, peut traverser les moyens espaceurs de part en part.

On entend ici par « recouvrir intégralement fonctionnellement », le fait que les moyens espaceurs 11 s'étendent sur toute la surface la face externe 1a, de la face intérieure 8a, de la face de la couche de détection colorimétrique 12 tournée vers l'espace intermédiaire 14, même si les moyens espaceurs 11 ne sont pas en contact avec la face en question, pourvu que tout éventuel débouché de non intégrité D sur la face en question soit empêché d'être occulté par suite des moyens espaceurs 11.

Quatre réalisations sont envisagées en ce qui concerne l'agencement de la structure des moyens espaceurs 11 en forme de couche par rapport à la première enveloppe / poche *stricto sensu* 1 et à la seconde enveloppe 8. Le cas échéant, ces quatre réalisations peuvent être combinées entre elles. Dans une première réalisation (figures 3B, 4B, 5B, 6B, 7B), les moyens espaceurs 11 en forme de couche recouvrent structurellement la face externe 1a de la paroi 3, 4 de la première enveloppe / poche *stricto sensu* 1.

Dans une deuxième réalisation (figures 3A, 4A, 5A, 7A), les moyens espaceurs 11 en forme de couche recouvrent structurellement la face intérieure 8a de la paroi 13 de la seconde enveloppe 8.

Dans une troisième réalisation (figures 3B, 6A), les moyens espaceurs 11 en forme de couche recouvrent structurellement la face de la couche de détection colorimétrique 12 tournée vers l'espace intermédiaire 14.

Dans une quatrième réalisation (figures 3C, 4C, 5C, 6C, 7C, 8A, 8B, les moyens espaceurs 11 en forme de couche sont placés dans l'espace intermédiaire 14.

On entend ici par « recouvrir structurellement », le fait que les moyens espaceurs 11 s'étendent au contact de la face externe 1a, de la face intérieure 8a, de la face de la couche de détection colorimétrique 12 tournée vers l'espace intermédiaire 14, y compris que les moyens espaceurs 11 fassent structurellement partie d'une paroi ou couche 3, 4, 13 et 12 de façon indissociable ou quasi indissociable ou de manière analogue.

Dans le cas où la première enveloppe / poche stricto *sensu* 1 est une poche 3D, les moyens espaceurs en forme de couche 11 recouvrent intégralement fonctionnellement et le cas échéant structurellement également, la face externe de l'intérieur de chacun des deux goussets 14. La « face externe » dont il est ici question s'entend comme la face externe 1a. On entend par « intérieur d'un gousset », la partie du gousset attenante au pli rentrant. Avec un gousset 4, il serait possible, en l'absence de moyens espaceurs tels que les moyens espaceurs 11, que les deux faces externes des deux parties du gousset 4 attenantes au pli rentrant viennent l'une contre l'autre et qu'un éventuel débouché de non intégrité D sur l'une des faces externes soit occulté par l'autre face externe. Grace à la présence des moyens espaceurs 11 dans chaque gousset 14, il n'y a aucun risque d'occultation car toute occultation est empêchée.

Selon une réalisation, les moyens espaceurs en forme de couche 11 recouvrent intégralement fonctionnellement, et le cas échéant structurellement, les premiers moyens d'introduction 7 et les premiers moyens d'extraction du produit ou dispositif biopharmaceutique, ainsi que les moyens d'introduction de gaz traceur 15 dont est éventuellement pourvue la première enveloppe / poche *stricto sensu* 1.

Les moyens espaceurs 11 en forme de couche présentent une souplesse telle qu'ils peuvent épouser la forme de la paroi ou couche 3, 4, 13 et 12, elle-même souple.

La poche 2 à témoin d'intégrité / non intégrité comprend ensuite un ou plusieurs gaz traceur choisi et un ou plusieurs détecteurs colorimétrique 12 de ce ou ces gaz traceurs, adapté(s) à celui-ci ou à ceux-ci.

Le gaz traceur se trouve soit dans le premier espace 6 de la première enveloppe / poche *stricto sensu* 1, soit dans l'espace intermédiaire 14 de la seconde enveloppe 8, à une pression partielle différente de la pression partielle qu'il a dans l'espace intermédiaire 14 ou du premier espace 6, respectivement. Cette définition inclut également le cas où l'un du premier espace 6 ou de l'espace intermédiaire 14 contient du gaz traceur et que l'autre de l'espace intermédiaire 14 ou du premier espace 6 ne contient pas ou substantiellement pas de gaz traceur, la pression partielle p étant nulle ou quasi nulle.

Le gaz traceur est introduit dans le premier espace 6 de la première enveloppe / poche *stricto sensu* 1 et/ou dans l'espace intermédiaire 14 de la seconde enveloppe 8 par des moyens d'introduction de gaz traceur 15, associés, respectivement, à la première enveloppe / poche stricto *sensu* 1 et/ou à la seconde enveloppe, à savoir, respectivement, à la paroi 3 et/ou paroi 13 correspondante. De tels moyens d'introduction de gaz traceur 15 peuvent être une valve, un port, un orifice obturable, ou analogue. Les moyens d'introduction de gaz traceur 15 peuvent être amenés à l'état ouvert pour l'introduction du gaz traceur, le cas échéant son évacuation si cela est souhaité. Ils peuvent également être amenés à l'état fermé, en dehors de ces circonstances et notamment lors du stockage et du transport de la poche 2 à témoin d'intégrité / non intégrité, lorsque se déroule le processus conduisant à identifier l'intégrité ou la non intégrité de la poche 2 à témoin d'intégrité / non intégrité.

Dans la poche 2 à témoin d'intégrité / non intégrité, les moyens d'introduction de gaz traceur 15, se trouvent à l'état fermé,

Un tel gaz traceur peut être choisi dans le groupe comprenant l'oxygène, le gaz carbonique, l'hélium, cette liste n'étant qu'exemplative et non limitative.

Un détecteur colorimétrique 12 de gaz traceur comprend une ou plusieurs couches continues de détection colorimétrique du gaz traceur.

Une telle couche continue de détection colorimétrique 12 est responsive, c'est-à-dire sensible, à la concentration du gaz traceur qui l'atteint en passant d'une première couleur à une seconde couleur différente de la première couleur, lorsque la concentration en gaz traceur l'atteignant passe une certaine valeur de transition. Une telle valeur de transition est connue pour chaque couple gaz traceur / couche continue de détection colorimétrique 12.

Une telle couche de détection colorimétrique 12 a pour caractéristique constructive structurelle ou fonctionnelle de faire partie intégrante d'une paroi 3, 4 de la première enveloppe / poche *stricto sensu* 1 et/ou d'une paroi 13 de la seconde enveloppe 8 et/ou des moyens espaceurs 11, selon les configurations envisagées. On entend par là que la couche de détection colorimétrique 12 n'est pas située au sein, c'est-à-dire au milieu du premier espace 6 ou du second espace 9, sauf dans la cas où elle fait partie intégrante de moyens espaceurs 11 eux-mêmes situés au sein de l'espace intermédiaire 14.

La couche de détection colorimétrique 12 présente une souplesse telle qu'elle peut épouser, autant qu'il est nécessaire, la forme de la paroi ou couche 3, 4, 13 et 11, elle-même souple.

Une telle couche de détection colorimétrique 12 forme un détecteur colorimétrique autonome, dépourvu de source d'énergie interne et de connexions extérieures.

La première couleur et la seconde couleur que peut prendre la couche de détection colorimétrique 12 doivent se différencier l'une de l'autre en sorte qu'il soit possible d'identifier si la couche de détection colorimétrique 12 est de la première couleur ou de la seconde couleur, sans ambigüité ou erreur. Une telle identification de couleur peut être humaine (par l'entremise d'un observateur extérieur) ou plus ou moins automatisée.

La poche 2 à témoin d'intégrité / non intégrité qui comprend les moyens espaceurs 11, le gaz traceur et la couche de détection colorimétrique 12 est apte à être aplatie ou substantiellement aplatie sur elle-même et ainsi aisément apte au stockage, au transport, à la manipulation.

Dans une réalisation (figures 3A, 3B, 3C, 4A, 4B, 4C, 5A, 5B, 5C, 6A, 6B, 6C, 8A, 8B), le gaz traceur est dans le premier espace 6 à une pression partielle P plus grande que la pression partielle p dans l'espace intermédiaire 14. Dans ce cas, la couche de détection colorimétrique 12 passe de la première couleur à la seconde couleur en réponse au passage de la concentration en gaz traceur qui l'atteint au-dessus de la valeur de transition.

Dans une autre réalisation (figures 7A, 7B, 7C), le gaz traceur est dans le premier espace 6 à une pression partielle p plus petite que la pression partielle P dans l'espace intermédiaire 14. Dans ce cas, la couche de détection colorimétrique 12 passe de la première couleur à la seconde couleur en réponse au passage de la concentration en gaz traceur qui l'atteint au-dessous de la valeur de transition.

Il est entendu que la présence et le choix du gaz traceur et la différence de pression P-p de celui-ci n'ont d'autre fonction que de faire changer de couleur la couche de détection colorimétrique 12, en fonction de la présence ou non d'un débouché de non intégrité sur la face externe 1a de la paroi 3, 4. La quantité et la pression du gaz traceur n'ont pas à être importantes.

La poche 2 à témoin d'intégrité / non intégrité qui comprend les deux enveloppes 1 et 8, les moyens espaceurs 11, le gaz traceur et la couche de détection colorimétrique 12 peut se présenter à plat pour le stockage, le transport, la manipulation.

Si la paroi 3, 4 (y compris les soudures 5) de la première enveloppe / poche *stricto sensu* 1 est intègre, le gaz traceur reste dans l'état initial dans lequel il a été introduit dans la poche 2 à témoin d'intégrité / non intégrité, la différence de pression initiale de part et d'autre de la première paroi 3, 4 étant maintenue.

Si la paroi 3, 4 n'est pas intègre, par suite d'un ou plusieurs passages T traversant la première enveloppe / poche *stricto sensu* 1, avec un ou plusieurs débouchés D sur la face externe 1a, la différence de pression initiale du gaz traceur diminue avec le temps, à la limite jusqu'à disparaître, le gaz traceur traversant la première enveloppe / poche *stricto sensu* 1 en passant par le ou les passages T et le ou les débouchés D, dans le sens allant de l'enceinte où la pression partielle est la plus grande à l'enceinte où la pression partielle est la plus petite. La pression partielle du gaz traceur dans l'enceinte où il a la pression partielle la plus grande diminue et, simultanément, la pression partielle du gaz traceur dans l'enceinte où il a la pression partielle la plus petite augmente.
La couche de détection colorimétrique 12 judicieusement disposée est alors atteinte par le gaz traceur dont la pression partielle et la concentration varient (diminution ou augmentation), de sorte que, selon la disposition, la concentration passe en-dessous ou au-dessus de la valeur de transition. Dans tous les cas, la couche de détection colorimétrique 12 change de couleur, passant de la première couleur à la seconde couleur en réponse au passage de la concentration en gaz traceur qui l'atteint en-dessous ou au-dessus de la valeur de transition.

Ainsi, si la couche de détection colorimétrique 12 est de la première couleur, il peut être conclu que la première enveloppe / poche *stricto sensu* 1 est intègre et donc est apte à recevoir et protéger le produit ou dispositif biopharmaceutique. Dans le cadre du processus de mise en oeuvre d'une poche *stricto sensu 1*, une telle première enveloppe / poche *stricto sensu* 1 peut être utilisée, c'est-à-dire tout d'abord extraite de la seconde enveloppe 8, puis ouverte pour y introduire le produit ou dispositif biopharmaceutique.

Si, au contraire, la couche de détection colorimétrique 12 est de la seconde couleur, il doit être conclu que la première enveloppe / poche *stricto sensu* 1 est non intègre et donc est inapte à recevoir et protéger le produit ou dispositif biopharmaceutique. Dans le cadre du processus de mise en oeuvre d'une poche *stricto sensu* 1, une telle première enveloppe / poche *stricto sensu* 1 ne doit pas être utilisée et en général elle est immédiatement écartée et détruite.

On choisit l'agencement de la poche 2 à témoin d'intégrité / non intégrité, en premier lieu, de manière que la couche de détection colorimétrique 12 ne puisse pas être atteinte par du gaz traceur se trouvant dans l'espace intermédiaire 14 ou dans le premier espace 6 avec une concentration passant la valeur de transition, avec la première enveloppe / poche *stricto sensu* 1 intègre.

On choisit l'agencement de la poche 2 à témoin d'intégrité / non intégrité, en second lieu, de manière que, et que la couche de détection colorimétrique 12 soit nécessairement atteinte par du gaz traceur se trouvant dans l'espace intermédiaire 14 ou dans le premier espace 6 avec une concentration passant la valeur de transition, avec une première enveloppe / poche *stricto sensu* 1 non intègre,

On choisit en conséquence la perméabilité ou l'imperméabilité au gaz traceur de la couche de détection colorimétrique 12 et des moyens espaceurs 11 pour que la couche de détection colorimétrique 12 soit effectivement atteinte par du gaz traceur se trouvant dans l'espace intermédiaire 14 ou dans le premier espace 6 avec une concentration passant la valeur de transition, avec une première enveloppe / poche *stricto sensu* 1 non intègre.

On décrit maintenant plusieurs agencements particuliers, de la poche 2 à témoin d'intégrité / non intégrité, les agencements particuliers décrits n'étant qu'exemplatifs et non limitatifs.

Selon un premier agencement illustré par les figures 3A, 3B et 3C, la couche de détection colorimétrique 12 est perméable au gaz traceur de sorte que le gaz traceur puisse l'atteindre si la première enveloppe / poche *stricto sensu* 1 non intègre, et elle fait partie intégrante de la paroi 3, 4 de la première enveloppe / poche *stricto sensu* 1 en étant située sur la face externe 1a de celle-ci et dans l'espace intermédiaire 14.

Selon un deuxième agencement illustré par les figures 4A, 4B et 4C, la couche de détection colorimétrique 12 est perméable au gaz traceur de sorte que le gaz traceur puisse l'atteindre si la première enveloppe / poche *stricto sensu* 1 est non intègre, et elle fait partie intégrante de la paroi 3, 4 de la première enveloppe / poche *stricto sensu* 1 en étant située sur la face interne de celle-ci et dans le premier espace 6.

Selon un troisième agencement illustré par les figures 5A, 5B et 5C, la couche de détection colorimétrique 12 est perméable au gaz traceur de sorte que le gaz traceur puisse l'atteindre si la première enveloppe / poche *stricto sensu* 1 est non intègre, et elle fait partie intégrante de la paroi 3, 4 de la première enveloppe / poche *stricto sensu* 1 en étant insérée dans l'épaisseur même de la paroi 3, 4, interposée entre sa face externe 1a et sa face interne 1b et interposée également entre le premier espace 6 et l'espace intermédiaire 14.

Selon un quatrième agencement illustré par les figures 6A, 6B et 6C, la couche de détection colorimétrique 12 fait partie intégrante de la paroi 13 de la seconde enveloppe 8 en étant située sur la face intérieure 8a et dans l'espace intermédiaire 14.

Selon un cinquième agencement illustré par les figures 7A, 7B et 7C, la couche de détection colorimétrique 12 fait partie intégrante de la paroi 13 en étant insérée dans l'épaisseur même de la paroi 13, interposée entre sa face intérieure 8a et sa face extérieure 8b. Dans ce cas, la couche 16 de la paroi 13 de la seconde enveloppe 13 adjacente d'un coté à la couche de détection colorimétrique 12 et de l'autre coté à la face intérieure 8a de la seconde enveloppe 8 est perméable au gaz traceur, de sorte que le gaz traceur puisse atteindre la couche de détection colorimétrique 12 de la première enveloppe / poche *stricto sensu* 1 non intègre.

Selon un sixième agencement illustré par la figure 8A, la couche de détection colorimétrique 12 fait partie intégrante d'une couche poreuse des moyens espaceurs 11 étant située vers la face intérieure 8a de la seconde enveloppe (8).

Selon un septième agencement illustré par la figure 8B, la couche de détection colorimétrique 12 fait partie intégrante d'une couche poreuse des moyens espaceurs 11 étant située vers la face externe 1ade la paroi 3, 4 de la première enveloppe / poche *stricto sensu* 1. Le gaz traceur atteint nécessairement la couche de détection colorimétrique 12, les moyens espaceurs 11 étant poreux c'est-à-dire perméables au gaz traceur.

Selon une variante non représentée des sixième et septième agencements, une couche de détection colorimétrique 12 et une couche poreuse des moyens espaceurs 11 sont structurellement confondues pour former une seule même couche, au lieu de former deux couches distinctes, mêmes associées l'une à l'autre.

Avec les dispositions constructives fonctionnelles ou structurelles décrites, il est possible d'identifier l'intégrité ou la non intégrité d'une poche *stricto sensu* 1 à tout moment souhaité après sa fabrication et au moins juste avant son utilisation projetée.

Cela peut être fait de façon sûre, aisée, rapide, sans la nécessité d'un dispositif dédié lourd ou complexe, ou d'opérations difficiles ou délicates, et sans la nécessité de devoir recourir à un personnel dédié au contrôle de l'intégrité, et sans la nécessité d'avoir à tester la poche de façon positive pour s'assurer de son intégrité, la non intégrité étant révélée de façon automatique, et donc sans la nécessité, lorsque la poche fait partie d'une pluralité de poches, d'avoir à tester chaque poche de façon positive.

Cette identification est réalisée par l'identification de la couleur de la couche de détection colorimétrique 12.

La seconde enveloppe 8 est conçue de manière à être apte à permettre d'identifier depuis l'extérieur d'elle-même si la couche de détection colorimétrique 12 est de la première couleur ou de la seconde couleur. Comme indiqué, une telle identification peut être réalisée de façon humaine par un observateur extérieur à la poche 2 à témoin d'intégrité / non intégrité. A cet effet, la seconde enveloppe 8 peut être, selon les réalisations, transparente ou translucide ou comporter une fenêtre transparente ou translucide pour observer la couleur du détecteur colorimétrique 12 situé en regard ou au voisinage de cette fenêtre.

Selon une réalisation possible, illustrée par la figure 1, il est prévu en outre un suremballage extérieur de protection 16 dans lequel est logée la poche 2 à témoin d'intégrité / non intégrité, la première enveloppe / poche *stricto sensu* 1 se trouvant alors dans la seconde enveloppe 8.

L'invention vise la poche 2 à témoin d'intégrité / non intégrité telle qu'elle a été décrite, et telle qu'elle a été réalisée.

L'invention vise également une telle poche 2 à témoin d'intégrité / non intégrité qui est intègre, la couche de détection colorimétrique 12 étant de la première couleur.

L'invention vise également une poche *stricto sensu* 1 intègre, destinée à recevoir et protéger un produit ou dispositif biopharmaceutique.

Une telle poche *stricto sensu* 1 intègre est constituée par la première enveloppe / poche *stricto sensu* 1 extraite d'une poche 2 intègre à témoin d'intégrité / non intégrité, c'est-à-dire dont la couche de détection colorimétrique 12 est de la première couleur.

L'invention vise également un procédé de réalisation d'une poche 2 à témoin d'intégrité / non intégrité telle qu'elle a été décrite.

A la base, on dispose d'une première enveloppe / poche *stricto sensu* 1, d'une seconde enveloppe 8 dont les seconds moyens d'introduction de la première enveloppe / poche *stricto sensu* 1 sont à l'état ouvert, de moyens espaceurs 11, de gaz traceur, la couche de détection colorimétrique 12 faisant partie intégrante d'une paroi 3, 4 de la première enveloppe / poche *stricto sensu* 1 et/ou d'une paroi 13 de la seconde enveloppe 8 et/ou des moyens espaceurs 11, comme il a été décrit.

Puis, on introduit la première enveloppe / poche *stricto sensu* 1 dans le second espace 9 de la seconde enveloppe 8 via les seconds moyens d'introduction à l'état ouvert, les moyens espaceurs 11 étant interposés entre la paroi 3, 4 et la paroi 13 de sorte à remplir leur fonction de non occlusion comme il a été décrit.

Puis, on introduit le gaz traceur, soit dans le premier espace (6) soit dans l'espace intermédiaire (14), à une pression partielle différente de celle de l'espace intermédiaire (14) ou du premier espace (6), respectivement, via les moyens d'introduction de gaz traceur 15, lesquels se trouvent alors à l'état ouvert. Puis on amène ces moyens d'introduction de gaz traceur 15 à l'état fermé.

On choisit l'agencement de la poche 2 à témoin d'intégrité / non intégrité pour que la couche de détection colorimétrique 12 ne puisse pas être atteinte ou au contraire soit nécessairement atteinte par du gaz traceur, comme il a été précédemment décrit et on choisit en conséquence la perméabilité ou l'imperméabilité au gaz traceur de la couche de détection colorimétrique 12 et des moyens espaceurs 11 pour que la couche de détection colorimétrique 12 soit effectivement atteinte par du gaz traceur lorsque cela est nécessaire.

*In fine,* la première enveloppe / poche *stricto sensu* 1 et la seconde enveloppe 8 sont fermées.

L'invention vise également un procédé d'obtention d'une poche *stricto sensu* 1 intègre et ainsi prête à recevoir et protéger un produit ou dispositif biopharmaceutique sans possible quelconque détérioration d'origine extérieure à la poche *stricto sensu* 1, telle qu'une contamination.

A la base, on dispose d'une poche 2 à témoin d'intégrité / non intégrité telle qu'elle a été décrite.

A tout moment souhaité après sa fabrication et au moins juste avant l'utilisation projetée de la poche *stricto sensu* 1, on identifie si la couche de détection colorimétrique 12 est de la première couleur ou de la seconde couleur.

Si la couche de détection colorimétrique 12 est de la première couleur, on amène les seconds moyens d'extraction de la première enveloppe / poche *stricto sensu* 1 dont est pourvue la seconde enveloppe 8 à l'état ouvert, on extrait la première enveloppe / poche *stricto sensu* 1 du second espace 9 de la seconde enveloppe 8, et on amène les premiers moyens 7 d'introduction du produit ou dispositif biopharmaceutique dont est pourvu la première enveloppe / poche *stricto sensu* 1 considérée comme intègre, à l'état ouvert en vue d'y placer le produit ou dispositif biopharmaceutique.

Si, au contraire, la couche de détection colorimétrique 12 est de la seconde couleur, on ne retient pas la première enveloppe / poche *stricto sensu* 1 en vue d'y placer le produit ou dispositif biopharmaceutique, la première enveloppe / poche *stricto sensu* 1 étant considérée comme non intègre.

Le processus de mise en oeuvre d'une poche *stricto sensu* 1 qui, si elle est intègre est destinée à recevoir et protéger un produit ou dispositif biopharmaceutique, comprenant sur un lieu de fabrication, des opérations initiales, sur un lieu d'utilisation, des opérations finales et sur un ou plusieurs lieux, des opérations intermédiaires est comme il est maintenant décrit.

Le processus comprend la réalisation, sur le lieu de fabrication, d'une poche à témoin d'intégrité / non intégrité 2 comprenant une poche *stricto sensu* 1, par le procédé de réalisation tel qu'il a été précédemment décrit.

Le processus comprend également le transport de la poche à témoin d'intégrité / non intégrité 2 du lieu de fabrication au lieu d'utilisation, moyennant le cas échéant des opérations de stockage et de manipulation.

Le processus comprend enfin, au moins sur le lieu d'utilisation et avant - tout spécialement juste avant - d'extraire la première enveloppe / poche *stricto sensu* 1 de la seconde enveloppe 8 en vue d'y placer le produit ou dispositif biopharmaceutique, l'identification que la couche de détection colorimétrique 12 est de la première couleur ou de la seconde couleur.

Comme il découle de la description qui précède, si la couche de détection colorimétrique 12 est identifiée de la première couleur, le processus le processus comprend alors l'utilisation de la poche *stricto sensu* 1 considérée comme intègre en vue d'y placer le produit ou dispositif biopharmaceutique pour le recevoir et le protéger.

Si, au contraire, la couche de détection colorimétrique 12 est identifiée de la seconde couleur, le processus consiste à ne pas utiliser la poche *stricto sensu* 1, celle-ci étant considérée comme non intègre en inapte à recevoir et protéger un produit ou dispositif biopharmaceutique. La poche *stricto sensu* 1 est alors écartée et le plus souvent détruite.

Le cas échéant, l'identification que la couche de détection colorimétrique 12 est de la première couleur ou de la seconde couleur est réalisée en temps masqué, lors des opérations intermédiaires de stockage, transport, manipulation.

## Revendications

1. Poche (2) à témoin d'intégrité / non intégrité en vue de recevoir et protéger un produit ou dispositif biopharmaceutique, comprenant :
▪ une première enveloppe (1), intérieure, souple, fermée, étanche, en matière plastique, comportant une paroi (3, 4) délimitant un premier espace (6), formant *stricto sensu* une poche (1) destinée à recevoir le produit ou dispositif biopharmaceutique, comportant des premiers moyens (7) d'introduction et des premiers moyens d'extraction du produit ou dispositif se trouvant à l'état fermé,
■ une seconde enveloppe (8), extérieure, fermée, étanche, en matière plastique, comportant une paroi (13) délimitant un second espace (9) dans lequel se trouve la première enveloppe / poche *stricto sensu* (1), comportant des seconds moyens d'introduction et des seconds moyens d'extraction de la première enveloppe / poche *stricto sensu* (1), se trouvant à l'état fermé, un espace intermédiaire (14) étant ainsi ménagé dans le second espace (9) hors de la première enveloppe / poche *stricto sensu* (1),
■ des moyens espaceurs (11) interposés entre la paroi (3, 4) et la paroi (13) aptes à ce que la face intérieure (8a) de la paroi (13) n'occulte pas un éventuel débouché de non intégrité sur la face externe (1a) de la paroi (3, 4),
■ au moins un gaz traceur, se trouvant soit dans le premier espace (6) soit dans l'espace intermédiaire (14), à une pression partielle différente de celle de l'espace intermédiaire (14) ou du premier espace (6), respectivement, introduit dans le premier espace (6) et/ou l'espace intermédiaire (14) par des moyens d'introduction de gaz traceur (15), se trouvant à l'état fermé,
■ au moins une couche continue de détection colorimétrique (12) du gaz traceur, responsive à la concentration du gaz traceur qui l'atteint en passant d'une première couleur à une seconde couleur différente, faisant partie intégrante d'une paroi (3, 4) de la première enveloppe / poche *stricto sensu* (1) et/ou d'une paroi (13) de la seconde enveloppe (8) et/ou des moyens espaceurs (11),
▪ la disposition des parois (3, 4, 13) de la première enveloppe / poche *stricto sensu* (1) et de la seconde enveloppe (8), des moyens espaceurs (11), du gaz traceur et de la couche de détection colorimétrique (12), d'une part, la perméabilité ou l'imperméabilité au gaz traceur de la couche de détection colorimétrique (12), et des moyens espaceurs (11), d'autre part, étant choisies pour que la couche de détection colorimétrique (12) ne puisse pas être atteinte par du gaz traceur se trouvant dans l'espace intermédiaire (14) ou dans le premier espace (6) avec une concentration passant la valeur de transition, avec la première enveloppe / poche *stricto sensu* (1) intègre, et que la couche de détection colorimétrique (12) soit nécessairement atteinte par du gaz traceur se trouvant dans l'espace intermédiaire (14) ou dans le premier espace (6) avec une concentration passant la valeur de transition, avec une première enveloppe / poche *stricto sensu* (1) non intègre,
telle que si la couche de détection colorimétrique (12) est identifiée de la première couleur, la première enveloppe / poche *stricto sensu* (1) est considérée comme intègre et apte à recevoir et protéger le produit ou dispositif biopharmaceutique, alors que si elle est identifiée de la seconde couleur, elle est considérée comme non intègre et inapte à recevoir et protéger le produit ou dispositif biopharmaceutique.

2. Poche (2) à témoin d'intégrité / non intégrité selon la revendication 1, **caractérisée par le fait que** les moyens espaceurs (11) comprennent au moins une couche poreuse recouvrant intégralement fonctionnellement la face externe (1a) de la paroi (3, 4) de la première enveloppe / poche *stricto sensu* (1) et/ou la face intérieure (8a) de la paroi (13) de la seconde enveloppe (8) et/ou la face de la couche de détection colorimétrique (12) tournée vers l'espace intermédiaire (14).

3. Poche (2) à témoin d'intégrité / non intégrité selon la revendication 2, **caractérisée par le fait que** les moyens espaceurs (11) comprennent au moins une couche poreuse qui soit recouvre structurellement la face externe (1a) de la paroi (3, 4) de la première enveloppe / poche *stricto sensu* (1) et/ou la face intérieure (8a) de la paroi (13) de la seconde enveloppe (8) et/ou la face de la couche de détection colorimétrique (12) tournée vers l'espace intermédiaire (14), soit est placée dans l'espace intermédiaire (14).

4. Poche (2) à témoin d'intégrité / non intégrité selon l'une quelconque des revendications 1 à 3, **caractérisée par le fait que** soit le gaz traceur est dans le premier espace (6) à une pression partielle plus grande que dans l'espace intermédiaire (14), la couche de détection colorimétrique (12) passant de la première couleur à la seconde couleur en réponse au passage de la concentration en gaz traceur qui l'atteint au-dessus d'une valeur de transition, soit le gaz traceur est dans le premier espace (6) à une pression partielle plus petite que dans l'espace intermédiaire (14), la couche de détection colorimétrique (12) passant de la première couleur à la seconde couleur en réponse au passage de la concentration en gaz traceur qui l'atteint au-dessous d'une valeur de transition.

5. Poche (2) à témoin d'intégrité / non intégrité selon l'une quelconque des revendications 1 à 4, **caractérisée par le fait qu'**elle comprend une des caractéristiques suivantes :
- une couche de détection colorimétrique (12) est perméable au gaz traceur et fait partie intégrante de la paroi (3, 4) de la première enveloppe / poche *stricto sensu* (1), étant soit située sur la face externe (1a) et dans l'espace intermédiaire (14), soit située sur la face interne (1b) et dans le premier espace (6), soit insérée dans la paroi (3, 4), interposée entre sa face externe (1a) et sa face interne (1b) et entre le premier espace (6) et l'espace intermédiaire (14) ;
- une couche de détection colorimétrique (12) fait partie intégrante de la paroi (13) de la seconde enveloppe (8), étant soit située sur la face intérieure (8a) et dans l'espace intermédiaire (14), soit insérée dans la paroi (13), interposée entre la face intérieure (8a) et sa face extérieure (8b), la couche (16) de la paroi (13) de la seconde enveloppe (13) adjacente d'un coté à la couche de détection colorimétrique (12) et de l'autre coté à la face intérieure (8a) étant alors perméable au gaz traceur ;
- une couche de détection colorimétrique (12) fait partie intégrante d'une couche poreuse des moyens espaceurs (11) étant soit située vers la face externe (1a) de la paroi (3, 4) de la première enveloppe / poche *stricto sensu* (1), soit située vers la face intérieure (8a) de la seconde enveloppe (8) ;
- une couche de détection colorimétrique (12) et une couche poreuse des moyens espaceurs (11) sont structurellement confondues pour former une seule même couche.

6. Poche (2) à témoin d'intégrité / non intégrité selon l'une quelconque des revendications 1 à 5, **caractérisée par le fait que** la première enveloppe / poche *stricto* sensu (1) est une poche 2D.

7. Poche (2) à témoin d'intégrité / non intégrité selon l'une quelconque des revendications 1 à 5, **caractérisée par le fait que** la première enveloppe / poche *stricto sensu* (1) est une poche 3D comportant deux goussets (4).

8. Poche (2) à témoin d'intégrité / non intégrité selon la revendication 7, **caractérisée par le fait que** les moyens espaceurs (11) comprennent au moins une couche poreuse recouvrant intégralement fonctionnellement, et le cas échéant structurellement, la face externe de l'intérieur des goussets (4).

9. Poche (2) à témoin d'intégrité / non intégrité selon l'une quelconque des revendications 1 à 8, **caractérisée par le fait**
**que** les moyens espaceurs (11) comprennent au moins une couche poreuse recouvrant intégralement fonctionnellement, et le cas échéant structurellement, les premiers moyens d'introduction et les premiers moyens d'extraction du produit ou dispositif biopharmaceutique et les moyens d'introduction de gaz traceur de la première enveloppe / poche *stricto sensu* (1).

10. Poche (2) à témoin d'intégrité / non intégrité selon l'une quelconque des revendications précédentes, **caractérisée par le fait**
**que** les moyens espaceurs (11) comprennent au moins une couche poreuse en tissu, en non tissé, en PE, en PP, en PTFE

11. Poche (2) à témoin d'intégrité / non intégrité selon l'une quelconque des revendications précédentes, **caractérisée par le fait**
**qu'**un gaz traceur est choisi dans le groupe comprenant l'oxygène, le gaz carbonique, l'hélium

12. Poche (2) à témoin d'intégrité / non intégrité selon l'une quelconque des revendications précédentes, **caractérisée par le fait**
**que** l'un du premier espace (6) ou de l'espace intermédiaire (14) contient du gaz traceur et que l'autre de l'espace intermédiaire (14) ou du premier espace (6) ne contient pas ou substantiellement pas de gaz traceur.

13. Poche (2) à témoin d'intégrité / non intégrité selon l'une quelconque des revendications précédentes, **caractérisée par** le fait
- que la seconde enveloppe (8) est apte à permettre d'identifier depuis l'extérieur d'elle-même si la couche de détection colorimétrique (12) est de la première couleur ou de la seconde couleur.

14. Poche (2) à témoin d'intégrité / non intégrité selon l'une quelconque des revendications précédentes, **caractérisée par le fait que** la seconde enveloppe (8) est souple.

15. Poche (2) à témoin d'intégrité / non intégrité selon l'une quelconque des revendications précédentes, **caractérisée par le fait qu'**
- elle comporte en outre un suremballage extérieur de protection (17) dans lequel est logée la seconde enveloppe (8), dans laquelle se trouve la première enveloppe / poche *stricto sensu* (1).

16. Poche (2) intègre à témoin d'intégrité / non intégrité constituée par une poche (2) à témoin d'intégrité / non intégrité selon l'une quelconque des revendications 1 à 15 dont la couche de détection colorimétrique (12) est de la première couleur.

17. Procédé de réalisation d'une poche (2) à témoin d'intégrité / non intégrité selon l'une quelconque des revendications 1 à 15, dans lequel :
▪ on dispose d'une première enveloppe / poche *stricto sensu* (1), d'une seconde enveloppe (8) dont les seconds moyens d'introduction de la première enveloppe / poche *stricto sensu* (1) sont à l'état ouvert, de moyens espaceurs (11), du gaz traceur, la couche continue de détection colorimétrique (12) faisant partie intégrante d'une paroi (3, 4) de la première enveloppe / poche *stricto sensu* (1) et/ou d'une paroi (13) de la seconde enveloppe (8) et/ou des moyens espaceurs (11),
▪ on introduit la première enveloppe / poche *stricto sensu* (1) dans le second espace (9) de la seconde enveloppe (8) via les seconds moyens d'introduction à l'état ouvert, les moyens espaceurs (11) étant interposés entre la paroi (3, 4) et la paroi (13) de sorte que la face intérieure (8a) de la paroi (13) n'occulte pas un éventuel débouché de non intégrité sur la face externe (1a) de la paroi (3, 4),
▪ on introduit le gaz traceur, soit dans le premier espace (6) soit dans l'espace intermédiaire (14), à une pression partielle différente de celle de l'espace intermédiaire (14) ou du premier espace (6), respectivement, via les moyens d'introduction de gaz traceur, se trouvant à l'état ouvert, puis on amène ces moyens d'introduction à l'état fermé,
▪ on choisit l'agencement pour que la couche de détection colorimétrique (12) ne puisse pas être atteinte ou au contraire soit nécessairement atteinte par du gaz traceur, comme exigé et on choisit en conséquence la perméabilité ou l'imperméabilité au gaz traceur de la couche de détection colorimétrique (12) et des moyens espaceurs (11) pour que la couche de détection colorimétrique (12) soit effectivement atteinte par du gaz traceur lorsque cela est nécessaire,
▪ la première enveloppe / poche *stricto sensu* (1) et la seconde enveloppe (8) étant fermées.

18. Procédé d'obtention d'une poche *stricto sensu* (1) intègre et prête à recevoir et protéger un produit ou dispositif biopharmaceutique, dans lequel :
▪ on dispose d'une poche (2) à témoin d'intégrité / non intégrité selon l'une quelconque des revendications 1 à 15,
▪ on identifie si la couche de détection colorimétrique (12) est de la première couleur ou de la seconde couleur,
▪ si la couche de détection colorimétrique (12) est identifiée de la première couleur :
∘ on amène les seconds moyens d'extraction de la première enveloppe / poche *stricto sensu* (1) dont est pourvue la seconde enveloppe (8) à l'état ouvert,
∘ on extrait la première enveloppe / poche *stricto sensu* (1) du second espace (9) de la seconde enveloppe (8),
∘ et on amène les premiers moyens (7) d'introduction du produit ou dispositif biopharmaceutique dont est pourvu la première enveloppe / poche *stricto sensu* (1) considérée comme intègre, à l'état ouvert en vue d'y placer le produit ou dispositif biopharmaceutique,
■ alors que si la couche de détection colorimétrique (12) est identifiée de la seconde couleur, on ne retient pas la première enveloppe / poche *stricto sensu* (1) en vue d'y placer le produit ou dispositif biopharmaceutique, la première enveloppe / poche *stricto sensu* (1) étant considérée comme non intègre.

19. Processus de mise en oeuvre d'une poche *stricto sensu* (1) qui, intègre, est destinée à recevoir et protéger un produit ou dispositif biopharmaceutique, comprenant :
▪ sur un lieu de fabrication, des opérations initiales consistant à fabriquer une poche *stricto sensu* (1),
▪ sur un lieu d'utilisation, des opérations finales consistant à utiliser la poche *stricto sensu* (1) en y plaçant le produit ou dispositif biopharmaceutique,
▪ sur un ou plusieurs lieux, des opérations intermédiaires de stockage, transport, manipulation, **caractérisé par** :
▪ la réalisation, sur le lieu de fabrication, d'une poche à témoin d'intégrité / non intégrité (2) comprenant une première enveloppe / poche *stricto sensu* (1), par le procédé de réalisation selon la revendication 17,
▪ le transport de la poche à témoin d'intégrité / non intégrité (2) du lieu de fabrication au lieu d'utilisation, moyennant le cas échéant des opérations de stockage et de manipulation,
▪ et, au moins sur le lieu d'utilisation et avant d'extraire la première enveloppe / poche *stricto sensu* (1) de la seconde enveloppe (8) en vue d'y placer le produit ou dispositif biopharmaceutique, l'identification que la couche de détection colorimétrique (12) est de la première couleur ou de la seconde couleur,
▪ si la couche de détection colorimétrique (12) est identifiée de la première couleur, l'utilisation de la première enveloppe / poche *stricto sensu* (1) considérée comme intègre en vue d'y placer le produit ou dispositif biopharmaceutique pour le recevoir et le protéger,
▪ alors que si la couche de détection colorimétrique (12) est identifiée de la seconde couleur, la non utilisation de la première enveloppe / poche *stricto sensu* (1) considérée comme non intègre en vue d'y placer le produit ou dispositif biopharmaceutique.

20. Processus selon la revendication 19, **caractérisé en ce que** l'identification que la couche de détection colorimétrique (12) est de la première couleur ou de la seconde couleur est réalisée en temps masqué lors des opérations intermédiaires de stockage, transport, manipulation.

21. Processus selon la revendication 19, **caractérisé en ce que** l'identification que la couche de détection colorimétrique (12) est de la première couleur ou de la seconde couleur est réalisée sur le lieu d'utilisation juste avant d'extraire la première enveloppe / poche *stricto sensu* (1) de la seconde enveloppe (8) en vue d'y placer le produit ou dispositif biopharmaceutique.

## Patentansprüche

1. Tasche (2) mit Unversehrtheits-/Versehrtheitskontrolleinrichtung zur Aufnahme und zum Schutz eines biopharmazeutischen Produkts oder einer biopharmazeutischen Vorrichtung, aufweisend:
▪ eine erste dichte, geschlossene, flexible, innere Hülle (1) aus Kunststoff, aufweisend eine Wand (3, 4), die einen ersten Raum (6) begrenzt, der im engeren Sinn eine Tasche (1) zur Aufnahme des biopharmazeutischen Produkts oder der biopharmazeutischen Vorrichtung bildet, aufweisend erste Mittel (7) zur Einführung und erste Mittel zur Entnahme des Produkts oder der Vorrichtung, die sich in geschlossenem Zustand befinden,
▪ eine zweite dichte, geschlossene äußere Hülle (8) aus Kunststoff, aufweisend eine Wand (13), die einen zweiten Raum (9) begrenzt, in dem sich die erste Hülle / Tasche im engeren Sinne (1) befindet, aufweisend zweite Mittel zur Einführung und zweite Mittel zur Entnahme der ersten Hülle / Tasche im engeren Sinn (1), die sich in geschlossenem Zustand befinden, einen Zwischenraum (14), der auf diese Weise in dem zweiten Raum (9) außerhalb der ersten Hülle / Tasche im engeren Sinn (1) angeordnet ist,
▪ Abstandsmittel (11), die zwischen der Wand (3, 4) und der Wand (13) angeordnet sind, so dass die Innenseite (8a) der Wand (13) ein mögliches Anzeichen einer Integritätsverletzung auf der Außenseite (1 a) der Wand (3, 4) nicht verdeckt,
▪ mindestens ein Tracergas, das sich entweder in dem ersten Raum (6) oder in dem Zwischenraum (14) befindet, mit einem Partialdruck, der von dem des Zwischenraums (14) bzw. des ersten Raums (6) verschieden ist, das in den ersten Raum (6) und/oder den Zwischenraum (14) mit Mitteln zur Einführung von Tracergas (15) eingeführt wird, die sich in geschlossenem Zustand befinden,
▪ mindestens eine durchgehende Schicht zum kolorimetrischen Nachweis (12) des Tracergases, die auf die Konzentration des Tracergases, das sie erreicht, reagiert, indem sie von einer ersten Farbe in eine andere, zweite Farbe übergeht, die integraler Bestandteil einer Wand (3, 4) der ersten Hülle / Tasche im engeren Sinn (1) und/oder einer Wand (13) der zweiten Hülle (8) und/oder der Abstandsmittel (11) ist,
▪ wobei die Anordnung der Wände (3, 4, 13) der ersten Hülle / Tasche im engeren Sinn (1) und der zweiten Hülle (8), der Abstandsmittel (11), des Tracergases und der kolorimetrischen Nachweisschicht (12) einerseits, die Durchlässigkeit oder die Undurchlässigkeit für das Tracergas der kolorimetrischen Nachweisschicht (12) und der Abstandsmittel (11) andererseits derart ausgewählt sind, dass die kolorimetrische Nachweisschicht (12) nicht von Tracergas, das sich in dem Zwischenraum (14) oder in dem ersten Raum (6) mit einer Konzentration befindet, die den Übergangswert übersteigt, erreicht werden kann, wenn die erste Hülle / Tasche im engeren Sinn (1) unversehrt ist, und dass die kolorimetrische Nachweisschicht (12) zwangsläufig von Tracergas, das sich in dem Zwischenraum (14) oder in dem ersten Raum (6) mit einer Konzentration befindet, die den Übergangswert übersteigt, erreicht wird, wenn eine erste Hülle / Tasche im engeren Sinn (1) versehrt ist,
so dass, wenn auf der kolorimetrischen Nachweisschicht (12) die erste Farbe identifiziert wird, die erste Hülle / Tasche im engeren Sinn (1) als unversehrt und als geeignet angesehen wird, das biopharmazeutische Produkt oder die biopharmazeutische Vorrichtung aufzunehmen und zu schützen, während wenn auf ihr die zweite Farbe identifiziert wird, sie als versehrt und als nicht geeignet angesehen wird, das biopharmazeutische Produkt oder die biopharmazeutische Vorrichtung aufzunehmen oder zu schützen.

2. Tasche (2) mit Unversehrtheits-/Versehrtheitskontrolleinrichtung nach Anspruch 1, **dadurch gekennzeichnet, dass** die Abstandsmittel (11) mindestens eine poröse Schicht aufweisen, die die Außenseite (1a) der Wand (3, 4) der ersten Hülle / Tasche im engeren Sinn (1) und/oder der Innenseite (8a) der Wand (13) der zweiten Hülle (8) und/oder die Seite der kolorimetrischen Nachweisschicht (12), die zum Zwischenraum (14) gedreht ist, funktionell vollständig bedeckt.

3. Tasche (2) mit Unversehrtheits-/Versehrtheitskontrolleinrichtung nach Anspruch 2, **dadurch gekennzeichnet, dass** die Abstandsmittel (11) mindestens eine poröse Schicht aufweisen, die entweder die Außenseite (1a) der Wand (3, 4) der ersten Hülle / Tasche im engeren Sinn (1) und/oder die Innenseite (8a) der Wand (13) der zweiten Hülle (8) und/oder die Seite der kolorimetrischen Nachweisschicht (12), die zum Zwischenraum (14) gedreht ist, strukturell bedeckt oder in dem Zwischenraum (14) angeordnet ist.

4. Tasche (2) mit Unversehrtheits-/Versehrtheitskontrolleinrichtung nach einem der Ansprüche 1 bis 3, **dadurch gekennzeichnet, dass** sich das Tracergas entweder in dem ersten Raum (6) befindet, mit einem Partialdruck, der größer ist als der in dem Zwischenraum (14), wobei die kolorimetrische Nachweisschicht (12) als Reaktion darauf, dass die Konzentration des Tracergases, das sie erreicht, einen Übergangswert übersteigt, von der ersten Farbe in die zweite Farbe übergeht, oder sich das Tracergas in dem ersten Raum (6) befindet, mit einem Partialdruck, der kleiner ist als im Zwischenraum (14), wobei die kolorimetrische Nachweisschicht (12) als Reaktion auf das Absinken der Konzentration des Tracergases, das sie erreicht, unter einen Übergangswert, von der ersten Farbe in die zweite Farbe übergeht.

5. Tasche (2) mit Unversehrtheits-/Versehrtheitskontrolleinrichtung nach einem der Ansprüche 1 bis 4, **dadurch gekennzeichnet, dass** sie eines der folgenden Merkmale aufweist:
- eine kolorimetrische Nachweisschicht (12) ist für das Tracergas durchlässig und integraler Bestandteil der Wand (3, 4) der ersten Hülle / Tasche im engeren Sinn (1), wobei sie entweder auf der Außenseite (1a) und in dem Zwischenraum (14) liegt, oder auf der Innenseite (1b) und in dem ersten Raum (6) liegt, oder in die Wand (3, 4), angeordnet zwischen ihrer Außenseite (1 a) und ihrer Innenseite (1 b), und zwischen dem ersten Raum (6) und dem Zwischenraum (14), eingefügt ist;
- eine kolorimetrische Nachweisschicht (12) ist integraler Bestandteil der Wand (13) der zweiten Hülle (8), die entweder auf der Innenseite (8a) und im Zwischenraum (14) liegt, oder in die Wand (13), angeordnet zwischen der Innenseite (8a) und ihrer Außenseite (8b), eingefügt ist, wobei die Schicht (16) der Wand (13) der zweiten Hülle (13), die einerseits zur kolorimetrischen Nachweisschicht (12) und andererseits zur Innenseite (8a) benachbart ist, dann für Tracergas durchlässig ist;
- eine kolorimetrische Nachweisschicht (12) ist integraler Bestandteil einer porösen Schicht der Abstandsmittel (11), die entweder in Richtung der Außenseite (1a) der Wand (3, 4) der ersten Hülle / Tasche im engeren Sinn (1) liegt, oder die in Richtung der Innenseite (8a) der zweiten Hülle (8) liegt;
- eine kolorimetrische Nachweisschicht (12) und eine poröse Schicht der Abstandsmittel (11) sind strukturell miteinander verschmolzen, um ein und dieselbe Schicht zu bilden.

6. Tasche (2) mit Unversehrtheits-/Versehrtheitskontrolleinrichtung nach einem der Ansprüche 1 bis 5, **dadurch gekennzeichnet, dass** die erste Hülle / Tasche im engeren Sinn (1) eine 2D-Tasche ist.

7. Tasche (2) mit Unversehrtheits-/Versehrtheitskontrolleinrichtung nach einem der Ansprüche 1 bis 5, **dadurch gekennzeichnet, dass** die erste Hülle / Tasche im engeren Sinn (1) eine 3D-Tasche ist, die zwei Falten (4) aufweist.

8. Tasche (2) mit Unversehrtheits-/Versehrtheitskontrolleinrichtung nach Anspruch 7, **dadurch gekennzeichnet, dass** die Abstandsmittel (11) mindestens eine poröse Schicht aufweisen, die funktionell und gegebenenfalls strukturell die Außenseite des Inneren der Falten (4) vollständig bedeckt.

9. Tasche (2) mit Unversehrtheits-/Versehrtheitskontrolleinrichtung nach einem der Ansprüche 1 bis 8, **dadurch gekennzeichnet,**
**dass** die Abstandsmittel (11) mindestens eine poröse Schicht aufweisen, die funktionell und gegebenenfalls strukturell die ersten Mittel zur Einführung und die ersten Mittel zur Entnahme des biopharmazeutischen Produkts oder der biopharmazeutischen Vorrichtung und die Mittel zur Einführung von Tracergas der ersten Hülle / Tasche im engeren Sinn (1) vollständig bedeckt.

10. Tasche (2) mit Unversehrtheits-/Versehrtheitskontrolleinrichtung nach einem der vorhergehenden Ansprüche, **dadurch gekennzeichnet,**
**dass** die Abstandsmittel (11) mindestens eine poröse Schicht aus Gewebe, aus Vlies, aus PE, aus PP, aus PTFE aufweisen.

11. Tasche (2) mit Unversehrtheits-/Versehrtheitskontrolleinrichtung nach einem der vorhergehenden Ansprüche, **dadurch gekennzeichnet,**
**dass** ein Tracergas ausgewählt ist aus der Gruppe, die Sauerstoff, Kohlendioxid, Helium aufweist.

12. Tasche (2) mit Unversehrtheits-/Versehrtheitskontrolleinrichtung nach einem der vorhergehenden Ansprüche, **dadurch gekennzeichnet,**
**dass** einer aus dem ersten Raum (6) oder dem Zwischenraum (14) Tracergas enthält und dass der andere aus dem Zwischenraum (14) oder dem ersten Raum (6) kein oder im Wesentlichen kein Tracergas enthält.

13. Tasche (2) mit Unversehrtheits-/Versehrtheitskontrolleinrichtung nach einem der vorhergehenden Ansprüche, **dadurch gekennzeichnet,**
- **dass** die zweite Hülle (8) geeignet ist, von außerhalb ihrer selbst festzustellen, ob die kolorimetrische Nachweisschicht (12) die erste Farbe oder die zweite Farbe aufweist.

14. Tasche (2) mit Unversehrtheits-/Versehrtheitskontrolleinrichtung nach einem der vorhergehenden Ansprüche, **dadurch gekennzeichnet,**
**dass** die zweite Hülle (8) flexibel ist.

15. Tasche (2) mit Unversehrtheits-/Versehrtheitskontrolleinrichtung nach einem der vorhergehenden Ansprüche, **dadurch gekennzeichnet,**
- **dass** sie ferner eine schützende äußere Umverpackung (17) aufweist, in der die zweite Hülle (8) angebracht ist, in der sich die erste Hülle / Tasche im engeren Sinn (1) befindet.

16. Unversehrte Tasche (2) mit Unversehrtheits-/Versehrtheitskontrolleinrichtung, die aus einer Tasche (2) mit Unversehrtheits-/Versehrtheitskontrolleinrichtung nach einem der Ansprüche 1 bis 15 besteht, bei der die kolorimetrische Nachweisschicht (12) die erste Farbe aufweist.

17. Verfahren zur Herstellung einer Tasche (2) mit Unversehrtheits-/Versehrtheitskontrolleinrichtung nach einem der Ansprüche 1 bis 15, wobei:
▪ eine erste Hülle / Tasche im engeren Sinn (1), eine zweite Hülle (8), bei der sich die zweiten Mittel zur Einführung der ersten Hülle / Tasche im engeren Sinn (1) in offenem Zustand befinden, Abstandsmittel (11), Tracergas, die durchgehende kolorimetrische Nachweisschicht (12), die integraler Bestandteil einer Wand (3, 4) der ersten Hülle / Tasche im engeren Sinn (1) und/oder einer Wand (13) der zweiten Hülle (8) und/oder der Abstandsmittel (11) ist, angeordnet werden,
▪ die erste Hülle / Tasche im engeren Sinn (1) in den zweiten Raum (9) der zweiten Hülle (8) über die zweiten Mittel zur Einführung in offenem Zustand eingeführt wird, wobei die Abstandsmittel (11) derart zwischen die Wand (3, 4) und die Wand (13) eingefügt sind, dass die Innenseite (8a) der Wand (13) ein mögliches Anzeichen einer Integritätsverletzung auf der Außenseite (1 a) der Wand (3, 4) nicht verdeckt,
▪ das Tracergas entweder in den ersten Raum (6) oder in den Zwischenraum (14) mit einem Partialdruck, der von dem des Zwischenraums (14) bzw. dem des ersten Raums (6) verschieden ist, über die Mittel zur Einführung von Tracergas eingeführt wird, die sich in offenem Zustand befinden, woraufhin diese Mittel zur Einführung in den geschlossenen Zustand überführt werden,
▪ die Anordnung derart ausgewählt wird, dass die kolorimetrische Nachweisschicht (12), wie gefordert, nicht von Tracergas erreicht werden kann oder im Gegenteil zwangsläufig erreicht wird, und wobei folglich die Durchlässigkeit oder die Undurchlässigkeit der kolorimetrischen Nachweisschicht (12) und der Abstandsmittel (11) für das Tracergas derart gewählt wird, dass die kolorimetrische Nachweisschicht (12) von Tracergas wirksam erreicht wird, wenn dies erforderlich ist,
▪ die erste Hülle / Tasche im engeren Sinn (1) und die zweite Hülle (8) geschlossen sind.

18. Verfahren zum Erhalt einer Tasche im engeren Sinn (1), die unversehrt und bereit ist, ein biopharmazeutisches Produkt oder eine biopharmazeutische Vorrichtung aufzunehmen und zu schützen, wobei:
▪ eine Tasche (2) mit Unversehrtheits-/Versehrtheitskontrolleinrichtung nach einem der Ansprüche 1 bis 15 angeordnet wird,
▪ identifiziert wird, ob die kolorimetrische Nachweisschicht (12) die erste Farbe oder die zweite Farbe aufweist,
▪ wenn bei der kolorimetrischen Nachweisschicht (12) die erste Farbe identifiziert wird:
o die zweiten Mittel zur Entnahme der ersten Hülle / Tasche im engeren Sinn (1), mit denen die zweite Hülle (8) versehen ist, in den offenen Zustand überführt werden,
o die erste Hülle / Tasche im engeren Sinn (1) aus dem zweiten Raum (9) der zweiten Hülle (8) entnommen wird,
∘ und die ersten Mittel (7) zur Einführung des biopharmazeutischen Produkts oder der biopharmazeutischen Vorrichtung, mit denen die erste Hülle / Tasche im engeren Sinn (1) ausgestattet ist, die als unversehrt angesehen wird, in den offenen Zustand überführt werden, um dort das biopharmazeutische Produkt oder die biopharmazeutische Vorrichtung anzuordnen,
▪ während, wenn bei der kolorimetrischen Nachweisschicht (12) die zweite Farbe identifiziert wird, die erste Hülle / Tasche im engeren Sinn (1) nicht verwendet wird, um dort das biopharmazeutische Produkt oder die biopharmazeutische Vorrichtung anzuordnen, die erste Hülle / Tasche im engeren Sinn (1) als versehrt angesehen wird.

19. Verfahren zur Umsetzung einer Tasche im engeren Sinn (1) die, unversehrt, zur Aufnahme und zum Schutz eines biopharmazeutischen Produkts oder einer biopharmazeutischen Vorrichtung bestimmt ist, aufweisend:
▪ an einem Fertigungsort, erste Arbeitsvorgänge, die darin bestehen, eine Tasche im engeren Sinn (1) herzustellen,
▪ an einem Verwendungsort, abschließende Arbeitsvorgänge, die darin bestehen, die Tasche im engeren Sinn (1) zu verwenden, indem in dieser das biopharmazeutische Produkt oder die biopharmazeutische Vorrichtung angeordnet wird,
▪ an einem oder mehreren Orten, dazwischen liegende Arbeitsvorgänge zur Lagerung, zum Transport, zur Handhabung, **gekennzeichnet durch**:
▪ die Herstellung, an dem Fertigungsort, einer Tasche mit Unversehrtheits-/Versehrtheitskontrolleinrichtung (2), aufweisend eine erste Hülle / Tasche im engeren Sinn (1), **durch** das Verfahren zur Herstellung nach Anspruch 17,
▪ den Transport der Tasche mit Unversehrtheits-/Versehrtheitskontrolleinrichtung (2) vom Fertigungsort zum Verwendungsort, mittels gegebenenfalls der Lagerungs- und Manipulationsvorgänge,
▪ und, mindestens am Verwendungsort und vor der Entnahme der ersten Hülle / Tasche im engeren Sinn (1) aus der zweiten Hülle (8), um dort das biopharmazeutische Produkt oder die biopharmazeutische Vorrichtung anzuordnen, die Identifikation, dass die kolorimetrische Nachweisschicht (12) die erste Farbe oder die zweite Farbe aufweist,
▪ wenn identifiziert wurde, dass die kolorimetrische Nachweisschicht (12) die erste Farbe aufweist, die Verwendung der ersten Hülle / Tasche im engeren Sinn (1), die als unversehrt angesehen wird, um darin das biopharmazeutische Produkt oder die biopharmazeutische Vorrichtung anzuordnen, um es/sie aufzunehmen und zu schützen,
▪ während, wenn identifiziert wird, dass die kolorimetrische Nachweisschicht (12) die zweite Farbe aufweist, die Nicht-Verwendung der ersten Hülle / Tasche im engeren Sinn (1), die als versehrt angesehen wird, um darin das biopharmazeutische Produkt oder die biopharmazeutische Vorrichtung anzuordnen, aufweist.

20. Verfahren nach Anspruch 19, **dadurch gekennzeichnet, dass** die Identifikation, ob die kolorimetrische Nachweisschicht (12) die erste Farbe oder die zweite Farbe aufweist, zeitparallel während den dazwischen liegenden Lagerungs-, Transport-, Manipulationsvorgängen durchgeführt wird.

21. Verfahren nach Anspruch 19, **dadurch gekennzeichnet, dass** die Identifikation, ob die kolorimetrische Nachweisschicht (12) die erste Farbe oder die zweite Farbe aufweist, am Ort der Verwendung unmittelbar vor der Entnahme der ersten Hülle / Tasche im engeren Sinn (1) aus der zweiten Hülle (8), um darin das biopharmazeutische Produkt oder die biopharmazeutische Vorrichtung anzuordnen, durchgeführt wird.

## Claims

1. Integrity/non-integrity indicator pouch (2) intended for receiving and protecting a biopharmaceutical product or device, comprising:
▪ a first, inner, envelope (1) which is flexible, closed, fluid-tight, and of plastic material, comprising a wall (3, 4) delimiting a first space (6), forming a pouch *stricto sensu* (1) intended to receive the biopharmaceutical product or device, comprising first introduction means (7) and first extraction means for respectively introducing/extracting the product or device, said means being in the closed state,
▪ a second, outer, envelope (8) which is closed, fluid-tight, and of plastic material, comprising a wall (13) delimiting a second space (9) in which is located the first envelope/pouch *stricto sensu* (1), comprising second introduction means and second extraction means for respectively introducing/extracting the second envelope/pouch *stricto sensu* (1), said means being in the closed state, an intermediate space (14) thus being arranged in the second space (9) outside the first envelope/pouch *stricto sensu* (1),
▪ spacing means (11) placed between the wall (3, 4) and the wall (13) such that the inner face (8a) of the wall (13) does not occlude any integrity-impacting opening in the outer face (1 a) of the wall (3, 4),
▪ at least one tracer gas, located either in the first space (6) or in the intermediate space (14), at a partial pressure that is different from that of the intermediate space (14) or of the first space (6), respectively, introduced into the first space (6) and/or the intermediate space (14) by tracer gas introduction means (15), now in the closed state,
▪ at least one continuous colorimetric detection layer (12) for detecting the tracer gas, responding to the concentration of tracer gas which reaches it by changing from a first color to a second and different color, being an integral part of a wall (3, 4) of the first envelope/pouch *stricto sensu* (1) and/or of a wall (13) of the second envelope (8) and/or of the spacer means (11),
▪ the arrangement of the walls (3, 4, 13) of the first envelope/pouch *stricto sensu* (1) and of the second envelope (8), the spacer means (11), the tracer gas, and the colorimetric detection layer (12), on the one hand, and the permeability or impermeability to the tracer gas of the colorimetric detection layer (12), and of the spacer means (11), on the other hand, being chosen so that the colorimetric detection layer (12) cannot be reached by tracer gas located in the intermediate space (14) or in the first space (6) at a concentration beyond the transition value, when the first envelope/pouch *stricto sensu* (1) is of satisfactory integrity, and so that the colorimetric detection layer (12) is in any case reached by tracer gas located in the intermediate space (14) or in the first space (6) at a concentration beyond the transition value, when the first envelope/pouch *stricto sensu* (1) is of unsatisfactory integrity,
such that if the colorimetric detection layer (12) is identified as being of the first color, the first envelope/pouch *stricto sensu* (1) is considered to be of satisfactory integrity and suitable for receiving and protecting the biopharmaceutical product or device, whereas if it is identified as being of the second color, it is considered to be of unsatisfactory integrity and unsuitable for receiving and protecting the biopharmaceutical product or device.

2. Integrity/non-integrity indicator pouch (2) according to claim 1, wherein the spacer means (11) comprise at least one porous layer fully and functionally covering the outer face (1 a) of the wall (3, 4) of the first envelope/pouch *stricto sensu* (1) and/or the inner face (8a) of the wall (13) of the second envelope (8) and/or the face of the colorimetric detection layer (12) facing the intermediate space (14).

3. Integrity/non-integrity indicator pouch (2) according to claim 2, wherein the spacer means (11) comprise at least one porous layer which either structurally covers the outer face (1 a) of the wall (3, 4) of the first envelope/pouch *stricto sensu* (1) and/or the inner face (8a) of the wall (13) of the second envelope (8) and/or the face of the colorimetric detection layer (12) facing the intermediate space (14), or is placed within the intermediate space (14).

4. Integrity/non-integrity indicator pouch (2) according to any of claims 1 to 3, wherein either the tracer gas is in the first space (6) at a higher partial pressure than in the intermediate space (14), the colorimetric detection layer (12) changing from the first color to the second color in response to the concentration of tracer gas which reaches it rising above a transition value, or the tracer gas is in the first space (6) at a lower partial pressure than in the intermediate space (14), the colorimetric detection layer (12) changing from the first color to the second color in response to the concentration of tracer gas which reaches it falling below a transition value.

5. Integrity/non-integrity indicator pouch (2) according to any of claims 1 to 4, comprising one of the following characteristics:
- a colorimetric detection layer (12) is permeable to the tracer gas and is an integral part of the wall (3, 4) of the first envelope/pouch *stricto sensu* (1), being located on the outer face (1a) and in the intermediate space (14), or located on the inner face (1b) and in the first space (6), or inserted within the wall (3, 4), between its outer face (1a) and its inner face (1b) and between the first space (6) and the intermediate space (14);
- a colorimetric detection layer (12) is an integral part of the wall (13) of the second envelope (8), being either located on the inner face (8a) and in the intermediate space (14), or inserted within the wall (13), between the inner face (8a) and its outer face (8b), the layer (16) of the wall (13) of the second envelope (13) adjacent on one side to the colorimetric detection layer (12) and on the other side to the inner face (8a) then being permeable to the tracer gas;
- a colorimetric detection layer (12) is an integral part of a porous layer of the spacer means (11) which is either located near the outer face (1a) of the wall (3, 4) of the first envelope/pouch *stricto sensu* (1), or is located near the inner face (8a) of the second envelope (8);
- a colorimetric detection layer (12) and a porous layer of the spacer means (11) are structurally combined to form a single layer.

6. Integrity/non-integrity indicator pouch (2) according to any of claims 1 to 5, wherein the first envelope/pouch *stricto sensu* (1) is a 2D pouch.

7. Integrity/non-integrity indicator pouch (2) according to any of claims 1 to 5, wherein the first envelope/pouch *stricto sensu* (1) is a 3D pouch comprising two gussets (4).

8. Integrity/non-integrity indicator pouch (2) according to claim 7, wherein the spacer means (11) comprise at least one porous layer completely and functionally, and where necessary structurally, covering the outer face of the inside of the gussets (4).

9. Integrity/non-integrity indicator pouch (2) according to any of claims 1 to 8, wherein the spacer means (11) comprise at least one porous layer which fully and functionally, and where necessary structurally, covers the first introduction means and the first extraction means for respectively introducing/extracting the biopharmaceutical product or device and the tracer gas introduction means of the first envelope/pouch *stricto sensu* (1).

10. Integrity/non-integrity indicator pouch (2) according to any of the preceding claims, wherein the spacer means (11) comprise at least one porous layer of fabric, nonwoven fabric, PE, PP, PTFE.

11. Integrity/non-integrity indicator pouch (2) according to any of the preceding claims, wherein a tracer gas is chosen from among the group comprising oxygen, carbon dioxide, and helium.

12. Integrity/non-integrity indicator pouch (2) according to any of the preceding claims, wherein one among the first space (6) or the intermediate space (14) contains tracer gas and the other among the intermediate space (14) or the first space (6) does not contain or substantially does not contain tracer gas.

13. Integrity/non-integrity indicator pouch (2) according to any of the preceding claims, wherein the second envelope (8) is such that it allows identifying from outside of itself whether the colorimetric detection layer (12) is of the first color or the second color.

14. Integrity/non-integrity indicator pouch (2) according to any of the preceding claims, wherein the second envelope (8) is flexible.

15. Integrity/non-integrity indicator pouch (2) according to any of the preceding claims, wherein it additionally comprises an outer protective packaging (17) which houses the second envelope (8) within which the first envelope/pouch *stricto sensu* (1) is located.

16. Integrity/non-integrity indicator pouch (2) of satisfactory integrity consisting of an integrity/non-integrity indicator pouch (2) according to any of claims 1 to 15 where the colorimetric detection layer (12) is of the first color.

17. Method for creating an integrity/non-integrity indicator pouch (2) according to any of claims 1 to 15, wherein:
▪ one provides a first envelope/pouch *stricto sensu* (1), a second envelope (8) in which the second introduction means for introducing the first envelope/pouch *stricto sensu* (1) are in the open state, spacer means (11), the tracer gas, the continuous colorimetric detection layer (12) which is an integral part of a wall (3, 4) of the first envelope/pouch *stricto sensu* (1) and/or of a wall (13) of the second envelope (8) and/or of the spacer means (11),
▪ the first envelope/pouch *stricto sensu* (1) is introduced into the second space (9) of the second envelope (8) via the second introduction means in the open state, the spacer means (11) being placed between the wall (3, 4) and the wall (13) such that the inner face (8a) of the wall (13) does not occlude any integrity-impacting opening on the outer face (1 a) of the wall (3, 4),
▪ the tracer gas is introduced into either the first space (6) or the intermediate space (14), at a partial pressure that is different from that of the intermediate space (14) or of the first space (6), respectively, via the tracer gas introduction means which are in the open state, then these introduction means are brought to the closed state,
▪ the arrangement is chosen so that the colorimetric detection layer (12) cannot be reached or conversely is necessarily reached by the tracer gas, as required, and the permeability or impermeability to the tracer gas of the colorimetric detection layer (12) and of the spacer means (11) is accordingly chosen so that the colorimetric detection layer (12) is in fact reached by the tracer gas when such is necessary,
▪ the first envelope/pouch *stricto sensu* (1) and the second envelope (8) being closed.

18. Method for obtaining a pouch *stricto sensu* (1) of satisfactory integrity and ready to receive and protect a biopharmaceutical product or device, wherein:
▪ one provides an integrity/non-integrity indicator pouch (2) according to any of claims 1 to 15,
▪ the colorimetric detection layer (12) is identified as being of the first color or the second color,
▪ if the colorimetric detection layer (12) is identified as being of the first color:
o the second extraction means, with which the second envelope (8) is equipped in order to extract the first envelope/pouch *stricto sensu* (1), are brought to the open state,
o the first envelope/pouch *stricto sensu* (1) is extracted from the second space (9) of the second envelope (8),
o and the first introduction means (7), with which the first envelope/pouch *stricto sensu* (1), considered to be of satisfactory integrity, is equipped in order to introduce the biopharmaceutical product or device, are brought to the open state in order to place the biopharmaceutical product or device within,
▪ whereas if the colorimetric detection layer (12) is identified as being of the second color, the first envelope/pouch *stricto sensu* (1) is considered to be of unsatisfactory integrity and is not used for placing a biopharmaceutical product or device within.

19. Process for making use of a pouch *stricto sensu* (1), which when of satisfactory integrity is intended for receiving and protecting a biopharmaceutical product or device, said process comprising:
▪ at a point of manufacture, initial operations consisting of manufacturing a pouch *stricto sensu* (1),
▪ at a point of use, final operations consisting of using the pouch *stricto sensu* (1) by placing the biopharmaceutical product or device within,
▪ intermediate storage, shipping, handling operations, at one or more locations,
**characterized by**:
▪ the creation by the method according to claim 17, at the point of manufacture, of an integrity/non-integrity indicator pouch (2) comprising a first envelope/pouch *stricto sensu* (1),
▪ the shipping of the integrity/non-integrity indicator pouch (2) from the point of manufacture to the point of use, including storage and handling operations when there are such,
▪ and, at least at the point of use and before extracting the first envelope/pouch *stricto sensu* (1) from the second envelope (8) in order to place the biopharmaceutical product or device within, the identification of whether the colorimetric detection layer (12) is of the first color or the second color,
▪ if the colorimetric detection layer (12) is identified as being of the first color, the first envelope/pouch *stricto sensu* (1) is considered to be of satisfactory integrity and is used for placing the biopharmaceutical product or device within in order to receive and protect it,
▪ whereas if the colorimetric detection layer (12) is identified as being of the second color, the first envelope/pouch *stricto sensu* (1) is considered to be of unsatisfactory integrity and is not used for placing the biopharmaceutical product or device within.

20. Process according to claim 19, wherein the identification of whether the colorimetric detection layer (12) is of the first color or of the second color is done concurrently with intermediate storage, shipping, or handling operations.

21. Process according to claim 19, wherein the identification of whether the colorimetric detection layer (12) is of the first color or of the second color is done at the point of use immediately prior to extracting the first envelope/pouch *stricto sensu* (1) from the second envelope (8) in order to place the biopharmaceutical product or device within.
